(19) **Europäisches Patentamt**
**European Patent Office**
**Office européen des brevets**

(11) **EP 4 440 732 B1**

(12) **EUROPEAN PATENT SPECIFICATION**

(45) Date of publication and mention
of the grant of the patent:
**04.02.2026 Bulletin 2026/06**

(21) Application number: **22830154.5**

(22) Date of filing: **02.12.2022**

(51) International Patent Classification (IPC):
***B01J 2/04*** *(2006.01)* ***A61K 9/50*** *(2006.01)*

(52) Cooperative Patent Classification (CPC):
**B01J 2/04; A61K 9/5063**

(86) International application number:
**PCT/EP2022/084162**

(87) International publication number:
**WO 2023/099713 (08.06.2023 Gazette 2023/23)**

(54) **PROCESS AND APPARATUS FOR THE MANUFACTURE OF SOLID MICROPARTICLES**

VERFAHREN UND VORRICHTUNG ZUR HERSTELLUNG VON FESTEN MIKROPARTIKELN

PROCÉDÉ ET APPAREIL POUR LA FABRICATION DE MICROPARTICULES SOLIDES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **03.12.2021 EP 21383090**

(43) Date of publication of application:
**09.10.2024 Bulletin 2024/41**

(73) Proprietor: **SiTec PharmaBio SL
08028 Barcelona (ES)**

(72) Inventors:
• **DANAN, Hana
08017 Barcelona (ES)**

• **ESPOSITO, Pierandrea
08017 Barcelona (ES)**
• **CARAMÉS BUENO, Jesús
08015 Barcelona (ES)**

(74) Representative: **ABG Intellectual Property Law,
S.L.
Avenida de Burgos, 16D
Edificio Euromor
28036 Madrid (ES)**

(56) References cited:
**US-A- 6 056 791      US-A1- 2005 206 023
US-B1- 6 375 873      US-B1- 6 630 121
US-B2- 7 087 197**

**Description**

**FIELD OF THE INVENTION**

The present invention relates to a process for the manufacture of spherical solid microparticles, as well as an apparatus for carrying out said process.

**BACKGROUND OF THE INVENTION**

In spray-freezing processes the solidification of the atomized product is obtained under a flow of cold gas ($CO_2$ or $N_2$), and the spraying and the size of the particles are mainly controlled by specific designs and performance of the nozzles; in such processes, small particles can be obtained only if a sufficiently high amount of energy is applied to the input. This is the case, for example, of the process disclosed in WO 2005/053656 A1, which uses a combined melt extrusion process followed by cryo-spraying, or in technologies based on the use of supercritical fluids (SCF) and highspeed rotating disc nozzles, including a first inlet for a solution or a suspension, a second separate inlet for an atomizing agent, such as supercritical (SC) $CO_2$, and a mixing device built-in the nozzle (EP1703965B1).

Many prior art processes need to mix in advance the product to be sprayed with supercritical or dense carbon dioxide, under different conditions and devices. In this sense, processes such as those disclosed in US 2005/051917 A1, describing particle production by $CO_2$-assisted nebulization with a bubble dryer, or in WO 2011/159218 A1 describing an apparatus and method for production of particles, describe a dynamic precipitation of substance from a fluid solution using supercritical $CO_2$ as antisolvent and spraying agent. WO 2007/028421, disclosing a gas-assisted atomization of dry particles by mixing product with SC-$CO_2$ and exposure to warm $N_2$ during atomization, also belongs to this category.

WO 99/22855 describes a method for cooling and atomizing liquid or pasty substances in which the substance is combined with liquid or supercritical carbon dioxide and the resulting mixture is expanded to ambient pressure. As a result of the expansion occurring during the combination, the liquid or pasty substance is atomized into fine particles and thereby cooled to a temperature below its solidification temperature. The liquid gas and the fluid substance are brought together at a mixing point just at the nozzles arrangement. Since the smaller the exchange surface, the higher the thermal energy density necessary to maintain flow, then the mixing temperature must be above the crystallization temperature of the liquid substance, otherwise solidification can occur at the nozzle, blocking the substance nozzle or the expansion nozzle used to relax the mixture. One clear disadvantage is that temperature-sensitive substances to be atomized are exposed to large amounts of heat and can therefore be damaged. Another possible disadvantage is that due to the thermal separation of the two material flows within the two-substances nozzle arrangement, and the external mixing that takes place at the mouth opening, expanding gas flow cannot be used so efficiently, which results in correspondingly larger particle diameters and particles not always spherical and with less reproducible characteristics.

WO 2005/049192 A1 seeks to counter this disadvantage by including the liquid or pasty stream of material to be atomized under high pressure gaseous carbon dioxide flow, mixed in a two-component (two-fluids) nozzle and then expanded, which leads to product particles. The cooling leading to the crystallization of the particles is then carried out by the expanding liquid carbon dioxide at a second nozzle which is arranged at a certain distance from the nozzle of the substance/carbon dioxide gas mixture. However, the main problem is that the thermal coupling of the product to the expanding liquid carbon dioxide is often inadequate and the cooling of the product is not always optimal.

More recently, EP3106217 B1 describes a specific device (3-fluids nozzle) and a method for atomization with simultaneous cooling of liquid or pasty substances, claiming high efficiency and in which the risk of freezing of a nozzle is minimized. The device is characterized in that the nozzle arrangement is a three-substance nozzle, through which feeds for the substance to be atomized, an expansion medium such as pressurized, liquified or supercritical gas and the atomizing gas are guided within a common housing and which open out at a common orifice, so that the feed for the atomizing gas runs between the feeds for the substance and the expansion medium and the feed line for the substance and the feed line for the atomizing gas are connected to a heating device. The spatial proximity at the exit of the three material flows from the three-component nozzle contributes to atomization and cooling of the product stream.

In technologies based on supercritical fluids, the product and the gas are sprayed from the same nozzle, which connects two lines, and mixes supercritical fluid and product, as for example in in US2005051917A1, or the product and the gas come directly into contact in a receiving chamber, or the product is mixed with the dense gas first, before spraying. In the case of water removal, the substance to be sprayed is often mixed with other miscible organic solvents, in usually unfavorable proportions (e.g. 1: 10 to 1:25), to be sprayed and extracted by SCF.

WO 2007/097626 A1 describes a process for the removal of water and the formation of dry solid particles, from compositions in aqueous liquids, said particles obtainable by eliminating water when in contact with a supercritical medium.

WO2007065716A2 reveal the use of SCF or dense gas as extraction medium, to eliminate water from aqueous liquids so to obtain colloidal suspensions, defining this property as "desolventing", which is always related to mixing products with supercritical, subcritical or dense $CO_2$ before $CO_2$ expansion and product spraying.

US 5,851,453 describes the possibility to eliminate water through supercritical fluid and form particles. The process for forming particles calls for the co-introduction, in the same particle formation container, of a supercritical fluid and the medium composition, and that supercritical conditions must be kept well above critical Tc and Pc to ensure simultaneous removal of the medium and its dispersion to form particles. In addition, the amount of water described in the composition is minimal, the aqueous vehicle being largely composed of an organic solvent miscible with water (e.g. ethanol: water 90: 10) and the concentration of drug dissolved in the vehicle is not greater than 0.5%.

US 6,056,791 describes a process defined as PGSS-drying (PGSS, Particles from Gas Saturated Solution), based on the contact between the product in solution and a stream of preheated supercritical $CO_2$, intimately mixed in a pressurized container. Once the solution is saturated with SC-$CO_2$, the mixture is sprayed into an expansion tower at room temperature and pressure, where $CO_2$ turns into gas, droplets are formed, and a powder is produced while the solvent (water) evaporates together with the gas exhausted. The invention is potentially applicable to obtain powders from drying of water-soluble materials. However, one limitation of the process is that the material and the active ingredients need to be dissolved in considerable amounts of water, which can reduce the drying capacity of the system. Another limitation is the necessity to mix and maintain the saturated mixture at quite high pressures to ensure that supercritical $CO_2$ conditions are maintained throughout the process.

EP2298286B 1 describes another methodology to produce micronized powders that comprise the following steps: mixing a feed substance or a mixture of feed products with water forming a water-containing mixture, bringing the water-containing mixture in contact with a supercritical or liquid pressurized medium at a two-component nozzle, and releasing the liquid pressurized medium simultaneously with the spraying of the water-containing mixture, whereby atomization and drying of the mixture is simultaneously achieved. However, the water removal efficiency of this process still needs to be improved.

US6630121 B1 describes a method of making fine dry particles of substances by forming a composition comprising a substance of interest and a supercritical or near critical fluid. The pressure on the composition is rapidly reduced whereby droplets are formed, and the droplets are then passed through a flow of heated gas.

There remains a need for a simple process, industrially scalable and economically viable to produce small, spherical microparticles, in particular overcoming the drawbacks of prior art processes and apparatuses, such as the use of complicated and difficult to operate nozzle designs (such as rotating disk nozzles or three-fluid nozzles), the use of supercritical fluids or carrying out process steps maintaining supercritical fluid conditions, use of organic solvents in combinations with supercritical fluids, use of high spraying temperatures, improving water removal efficiency and improving microspheronization results.

## SUMMARY OF THE INVENTION

The inventors have surprisingly found a simple process and apparatus that allows obtaining small, spherical microparticles comprising biologically active molecules, as shown in the examples. Also, the particles obtained when using the process and apparatus of the invention are spherical and have a narrower particle size distribution, as shown in the examples. Further, as also shown in the examples, the process and apparatus of the invention offers a superior grade capacity to eliminate water at low temperatures. Also, the process can be carried out using relatively low-cost equipment since there is no need to maintain any part of the equipment at supercritical pressure and temperature conditions and it also avoid the use of complex nozzle designs, it can be performed at any scale (laboratory, pilot or industrial scale).

Thus, in the first aspect, the present invention relates to a process for the manufacture of solid microparticles comprising:

a) providing a fluid mixture comprising one or more molecules of interest one or more compounds selected from the group consisting of lipids, lipid-polymer compounds and polymers, and optionally water,
b) mixing the fluid mixture of step a) with a desiccating gas and/or plasticizing gas to provide a dispersion of the fluid mixture of step a) and the desiccating gas and/or plasticizing gas,
c) ejecting the dispersion obtained in step b) through a nozzle thereby creating a capillary or preatomized flow, and
d) contacting the capillary or preatomized flow obtained in step c) with an expanding liquid or dense $CO_2$ swirling or spiraling flow to obtain the solid microparticles.

In a second aspect, the invention relates to an apparatus for performing the process according to the first aspect, the apparatus comprising:

i) a first vessel for providing the fluid mixture comprising the one or more molecules of interest, the one or more compounds selected from the group consisting of lipids, lipid-polymer compounds and polymers, and optionally water,
ii) a source of the desiccating gas and/or plasticizing gas,

iii) a device for mixing the fluid mixture comprising the one or more molecules of interest, one or more compounds selected from the group consisting of lipids, lipid-polymer compounds and polymers, and optionally water, with the desiccating gas and/or plasticizing gas to provide the dispersion of the fluid mixture and the desiccating gas and/or plasticizing gas,

iv) means for conveying the fluid mixture of the first vessel i) to the device iii),

v) means for conveying the desiccating gas and/or plasticizing gas from the source ii) to the device iii),

vi) a second vessel for microparticles formation,

vii) one or more nozzles for creating a capillary or preatomized flow of the dispersion of the fluid mixture and the desiccating gas and/or plasticizing gas located in the upper part of the second vessel vi),

viii) means for conveying the dispersion of the fluid mixture and the desiccating gas and/or plasticizing gas of the device iii) to the one or more nozzles vii),

ix) a source of the liquid or dense $CO_2$,

x) a nozzle for creating a swirling or spiraling flow of liquid or dense $CO_2$ located in the upper part of the second vessel vi),

xi) means for conveying the liquid or dense $CO_2$ from the source ix) to the nozzle x), and wherein the one or more nozzles vii) are positioned at a distance with respect to nozzle x) of from 0.1 to 500 mm, preferably from 5 to 50 mm.

## DESCRIPTION OF THE FIGURES

Figure 1 shows a general scheme of an apparatus according to the invention.

Figure 2 shows a spraying apparatus device according to the invention, wherein 1 is a nozzle plate, 2 is a $CO_2$ nozzle with swirling tip, 3 is a product nozzle with capillary flow, 4a is a $CO_2$ line valve, 4b is a product line valve, 4c is a preatomization line valve, 5 is a static mixer, 6a is a product line heat exchanger, 6b is a preatomization line heat exchanger, 7 is a $CO_2$ bottle/reservoir, 8 is a product vessel and 9 is a nitrogen bottle/reservoir.

Figure 3 shows a schematic representation of particle formation within a swirling flow (left) or a conic flow (right) of expanding, liquid $CO_2$.

Figure 4 shows a schematic representation of liquid $CO_2$ nozzle (nozzle x) and product nozzle (nozzle vii) at an angle of $\alpha$.

Figure 5 shows a schematic representation of a static mixer and a product nozzle (nozzle vii)) in an apparatus according to the invention.

Figure 6 shows scanning electron microscopy images evidencing the shape and morphology of microparticles obtained according to Comparative Example 1 (left) and Comparative Example 2 (right). Scale bar on images: 100 $\mu$m, magnification 100x. Experimental conditions: probe current SEI 5kV; Focal depth: WD11mm SS30, 100x (Left), WD13mm SS50, 100x (right).

Figure 7 shows optical microscopy images of Lot SMX03 microparticles as described in the example 4. Left: 100x magnification; (1 major tick on image ruler =100 $\mu$m). Right: 400x (1major tick=25$\mu$m).

Figure 8 shows SEM images of microparticles prepared as described in the example 4. Scale bars: left image: 50 $\mu$m (500x magnification); right image: 100 $\mu$m, (200x magnification). Experimental conditions: SEI 5kV, WD11mmSS40, x500 (Left), SEI 5kV, WD11mmSS40, x200 (right).

Figure 9 shows optical microscopy (100x; 1 major tick =100$\mu$m), at left, and SEM image (scale bar: 100 $\mu$m, 120x magnification), at right, of microparticles produced according to comparative example 5. SEM Experimental conditions: SEI 10kV, WD11mmSS30, x120.

Figure 10 shows the residual water (% weight on solid) in different compositions of microspheres vs. initial water content in sprayed formulations of Example 8. Comparison of lipid-polyoxyl esters/glyceril di-behenate excipients ratios: squares 10:90; diamonds 15:85; triangles 30:70.

Figure 11 shows optical microscopy images of DE007, Comparative Example 15 (left), and DE 052, Comparative Example 16 (right). (Magnification 100x; 1 major tick =100$\mu$m)

Figure 12 shows optical microscopy images of DE 041, Comparative Example 18 (left), and DE 052, Comparative Example 19 (right). (Magnification 100x; 1 major tick =100$\mu$m)

Figure 13 shows optical microscopy of microspheres powders described in Table 11 (100x magnification, 1 major tick =100$\mu$m, and 400x magnification, 1 major tick =25$\mu$m).

Figure 14 shows microparticles containing curcuma extract obtained in example 24a, (left) and those obtained in comparative example 24b (right). Scale bars: left image: 200 $\mu$m (70x magnification); right image: 100 $\mu$m, (100x magnification). Experimental conditions: SEI 20kV, WD12mmSS50, x70 (Left), SEI 4kV, WD11mmSS50, x100 (right).

Figure 15 shows representative SEM images at different magnifications showing structural details of lycopene-containing microparticles, LY017, Example 25. Scale bars: left image: 500 $\mu$m (40x magnification); right image: 100 $\mu$m, (250x magnification). Experimental conditions: SEI 10kV, WD11mmSS50, x500 (Left), SEI 10kV, WD11mmSS50, x250 (right).

Figure 16 shows optical microscopy (100x magnification, 1 major tick =100$\mu$m), at left, and SEM images (scale bar 100 $\mu$m (100x magnification), at right, of microspheres from wine lees extracts produced according to example 26. SEM experimental conditions: SEI 10kV, WD11mmSS50, x100

Figure 17 shows the circularity distribution of 4 different microspheres samples: SMX003 and SMX004, from example 4; DE042 from example 22 and DE054 from comparative example 19.

## DETAILED DESCRIPTION OF THE INVENTION

Process of the invention

In the first aspect, the present invention relates to a process for the manufacture of solid microparticles comprising:

a) providing a fluid mixture comprising one or more molecules of interest one or more compounds selected from the group consisting of lipids, lipid-polymer compounds and polymers, and optionally water,
b) mixing the fluid mixture of step a) with a desiccating gas and/or plasticizing gas to provide a dispersion of the fluid mixture of step a) and the desiccating gas and/or plasticizing gas,
c) ejecting the dispersion obtained in step b) through a nozzle thereby creating a capillary or preatomized flow, and
d) contacting the capillary or preatomized flow obtained in step c) with an expanding liquid or dense $CO_2$ swirling or spiraling flow to obtain the solid microparticles.

In the context of the present invention, the term "microparticles" is to be understood as particles having a mean size of less than 300 $\mu$m, preferably a mean size comprised between 0.5 and 200 $\mu$m, more preferably between 1 and 100 $\mu$m. The mean size of the particles can be determined by different techniques of dimensional analysis. Preferably, Laser Light Diffraction method (ref. 2.9.31. European Pharmacopeia 6.0, pg. 311-314) is used, as disclosed in all the examples except examples 27 and 28. Briefly, samples were prepared suspending the microparticles in water, containing 0.1 to 0.5% of Tween 80, and sonicated for 30 seconds. An aliquot of the samples was subsequently placed in the reading cell of the particles size analyzer (Malvern Mastersizer 3000). Alternatively, particles size may be measured with Static Automatic Imaging technique - Morphologi 4 (ref. https://www.malvernpanalytical.com/en), as in examples 27 and 28, where the instrument achieves a physical separation of particles of dry powders and captures images of individual particles by scanning the sample underneath the microscope high resolution optics

The term "solid microparticles" refers to microparticles as defined herein wherein the composition of the microparticle is in solid form, i.e. is not a fluid. Preferably, the solid microparticles of the invention have a matrix configuration, i.e. the composition of the microparticle is homogeneous throughout the microparticles, as opposed to core-shell microparticles or hollow microparticles.

The microparticles obtained with the process of the present invention are substantially spherical, i.e. microspheres. The sphericity of the microparticles can be assessed as described in the examples, based on their circularity (C) parameter, calculated by analyzing the results from Static Automatic Imaging technique. The circularity is defined as the degree to which a visualized particle is similar to a circle, taking into consideration the smoothness of the perimeter. Therefore, circularity is a measurement of both the particle form and its roughness. Thus, the closest to a perfectly round, smooth circle a particle is, the closest the circularity value is to 1.0. Circularity is defined as:

$$C = \sqrt{\frac{4\pi A}{P^2}}$$

where A is the area of the observed particle, and P is its perimeter.

Preferably, the circularity of the microparticles obtained with the process of the present invention is greater than 0.7, preferably greater than 0.75, more preferably greater than 0.8, more preferably greater than 0.85, even more preferably greater than 0.90.

The first step of the process of the invention, step a), is providing a fluid mixture comprising one or more molecules of interest, one or more compounds selected from the group consisting of lipids, lipid-polymer compounds and polymers, and optionally water.

The term "fluid mixture" refers to a mixture in fluid form, which is a mixture or material that has no fixed shape and that continuously deforms under an applied shear stress or external force, i.e. it has the ability to take on the shape of the container when poured.

The "molecule of interest" refers to any biologically active molecule, such as nutritionally, pharmacologically or cosmetically active molecules.

Examples of nutritionally active molecules are carotenoids such as astaxanthin, lycopene, lutein, zeaxanthin; other antioxidants such as curcumin and curcuminoids, ferulic acid esters such as gamma-oryzanol, polyphenols like resveratrol and quercetin; polyunsaturated fatty acids (PUFAs) and fatty esters, especially but not exclusively omega-3 fatty acids like eicosapentaenoic acid (EPA) and docosahexaenoic (DHA); proteins and peptides from vegetable origin such as pea, soy, wheat, proteinaceous materials from other origin such as lactoferrin, collagen, insect proteins; vitamins C, D, E and in general all hydrosoluble and liposoluble vitamins and vitamin complexes; mono, oligo and polysaccharides such as e.g. natural fibers, chitosan, alginate, hyaluronic acid, other natural extracts with properties as nutritional supplements, for instance vegetable extracts from blackberries, blueberries, cranberries, pomegranate grapes and wine lees, gac fruit (*momordica cochinchinensis*), extracts from microalgae such *haematococcus pluvialis, spiruline ,* and in general all natural extracts that present a nutritional value and properties.

Examples of pharmacologically active molecules are any pharmacologically active molecule that is hydrophilic, lipophilic or amphiphilic. Examples of hydrophilic or hydrosoluble molecules are small organic molecules such as antibiotic clindamycin phosphate; anti emetic as metoclopramide; sodium ibandronate, and other bisphosphonates for treatment of ostheoporosis; DPP-IV inhibitors like e.g. sitagliptin, saxagliptin, linagliptin, and alogliptin; more in general all molecules pertaining to BCS classification classes I and III; peptide molecules such as for instance GLP-1 receptor agonists like liraglutide, semaglutide, GLP-2 receptor agonists like teduglutide, parathyroides hormones such as teriparatide, other peptides molecules such as leuprolide and octreotide; oliogonucleosides such e.g. inotersen sodium and nusinersen sodium; high molecular weight therapeutic proteins; enzymes such as pancreatin, modified proteins such as pegylated proteins or peptides.

Examples of lipophilic or liposoluble molecules are: antinflammatory drugs such as COX-2 inhibitor celecoxib, cannabinoids such as e.g. tetrahidrocanabinol (THC) cannabidiol (CBD), cannabidiolic acid (CBDA); kinase inhibitors, such as sunitinib, pazopanib, nilotinib, olaparib, cryzotinib, and other molecules of kinase inhibitors class; retinoids such as retinol, tretinoin, or adapalene; antiepilectics such as e.g lacosamide; antifungals belonging to echinocandins class, such as echinocandin and caspofungin, antioxidants such as lycopene, astaxanthin, curcumin, and in general all the lipophilic or liposoluble molecules pertaining to the Biopharmaceutical Classification classes II and IV.

Examples of cosmetically active molecules are hyaluronic acid, carotenoids, antioxidants, gamma orizanoles, phospholipids, ceramides, and in general all molecules from natural or synthetic source that can have suitable cosmetic application

In a preferred embodiment, the molecule of interest is hygroscopic, such as for example pharmacologically actives like clindamycin phosphate, or hygroscopic excipients.

The term "hygroscopic" refers to a compound that adsorbs and/or absorbs water from the surrounding environment. The hygroscopicity of a compound may be assessed by determining the increase in mass of a sample upon storage at 25 °C and 80% RH for 24 h. A compound that picks up more than 2% by mass is considered as being hygroscopic.

The one or more molecules of interest may be present in the fluid mixture of step a) in 0.1 to 99 wt%, preferably 1 to 80 wt%, more preferably 10 to y 70 wt%, wherein the weight percentages (wt%) are expressed with respect to the total weight of the fluid mixture of step a).

The expression "one or more" means, for example, 1, 2, 3, 4, 5, 6, 7, 8, 9 or 10 items.

The term "lipid" refers to a fatty, waxy, or oily compound that is soluble in organic solvents and insoluble in polar solvents such as water. Soluble refers to a compound that dissolves in an amount of 1 gram in less than 10 mL of the corresponding solvent at 25°C. Insoluble refers to a compound that does not dissolve in an amount of 1 gram in at least 10 mL of the corresponding solvent at 25 °C. Lipids include fats, oils, phospholipids, waxes and steroids. Examples of lipids are fatty acids, mono-, di- and triesters of glycerol and fatty acids, such as stearic acid, glyceryl monostearate, glyceryl tristearate, glyceryl dibehenate, medium chain triglycerides (MCT) such as caprylic/capric triglyceride.

The term "fatty acid" refers to carboxylic acids having an aliphatic chain which can be saturated or unsaturated (for example having 1, 2, 3, 4, 5 or 6 unsaturations, preferably 2, 3, 4, 5 or 6 double bonds). The number of carbon atoms, including that of the carboxylic group is usually from 6 to 26. Examples of fatty acids are caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, linolenic acid, arachidonic acid, eicosapentaenoic acid, erucic acid, docosahexaenoic acid.

The term "polymer" refers to active molecules or excipients of polymeric nature, of synthetic or natural origin. Examples of synthetic polymers that can be used in the invention are polyethylene glycols (PEGs), Poloxamers (PEO-PPO copolymers), polyvinyl caprolactam-polyvinyl acetate- polyethylene glycol copolymer (Soluplus®), polyvinilpirrolidone, polymethyl methacrylates (Eudragit®). Examples of natural polymers that can be used in the invention are alginate, chitosan, xanthan, guar gum, locust bean gums, hyaluronic acid. Likewise, cyclic oligosaccharides (oligomers such as beta-cyclodextrins, hydroxypropyl-beta cyclodextrin, sulfo-butyl-ether beta cyclodextrin, and in general all molecules and derivatives of cyclodextrin class.

The term "lipid-polymer compound" refers to molecules combining a lipid part and a polymeric part joined by covalent bonds, for instance lipid-polymer esters, such as polyoxylglycerides, TPGS, stearoyl or lauroyl-macrogol-polyoxyl-glycerides (Gelucire 50/13 or Gelucire 44/14). The term "lipid-polymer compound" also refers to composite materials comprising a lipid part and a polymeric part bound by non-covalent bonds, such as for instance eutectic compositions, co-amorphous forms and cocrystals, detectable by any of the analytical methods known to the skilled in the art.

Eutectic mixtures are defined as a mixture of two or more phases at a particular composition of materials that have the lowest melting temperature at which the phases will simultaneously crystallize. For example, eutectic blends comprising a lipid fraction composed of fatty acids, such as stearic acid, saturated mono-, di- or tri- glycerides such as glyceryl stearates, palmitates, behenates, or unsaturated such as oleates, linoleates, caprylates, and a polymer such as, for example, polyethylene glycol, poloxamer, polymethacrylates, or any other blend of a lipid and a polymer having a physical molecular arrangement originating a eutectic.

A co-amorphous solid is defined as a single phase amorphous solid system composed by two or more molecular components defined as co-formers. Based on the selection of co-formers, co-amorphous systems can be formed by the arrangement of two or more active molecules, one active molecule and one or more excipients, or by two or more excipients such as lipid-polymer compounds described herein, alone or in combination with an active molecule.

Cocrystalline forms are defined as solids that are crystalline materials composed of two or more molecules in the same crystal lattice.

A solid dispersion is defined as a eutectic mixture whereas the solid phases constituting the eutectic each contain only one component such that the system can be regarded as an intimate mixture of one component in the other.

A solid solution is a solid dispersion in which a solid solute is dissolved in a solid at molecular level.

Preferably, the lipid-polymer compounds are selected from the group consisting of polyoxylglycerides (i.e. mono-, di and triglycerides of polyethoxylated fatty acids, such as stearoyl polyoxyl glycerides, caprjyloyl polyoxyl glycerides, D-α-tocopheryl polyethylene glycol succinate (TPGS) and mixtures thereof.

In a particular embodiment, the fluid mixture of step a) further comprises glycerin and/or diethylene glycol monoethyl-ether (Transcutol).

In a preferred embodiment, at least one of the one or more compounds selected from the group consisting of lipids, lipid-polymer compounds and polymers is hygroscopic, such as, for example, stearoyl or lauroyl-macrogol-polyoxyl-glycerides (Gelucire 50/13 or Gelucire 44/14) polyethylene glycols, poloxamers (PEO-PPO copolymers), polysaccharides such as alginate, chitosan, xanthan gum, guar gum, cyclodextrins, sucro-esters such as e.g. sucrose ester of palmitic/stearic acid.

Preferably, the one or more lipids, lipid-polymer compounds and polymers of the fluid mixture of step a) is selected from the group consisting of mono-, di- and tri-glycerides of fatty acids, polyoxylglycerides, TPGS and mixtures thereof.

The one or more compounds selected from the group consisting of lipids, lipid-polymer compounds and polymers may be present in the fluid mixture of step a) in 0.1 to 99.9 wt%, preferably 20 to 99 wt%, more preferably 30 to 90 wt%, wherein the weight percentages (wt%) are expressed with respect to the total weight of the fluid mixture of step a).

The fluid mixture of step a) optionally comprises water, i.e. water may be present or absent. In one embodiment the composition comprises water. In another embodiment the composition is devoid of water (i.e. the water content as determined by Karl Fisher is lower than 1 wt%, preferably lower than 0.5 wt%, more preferably lower than 0.01 wt%).

When the fluid mixture of step a) comprises water, the amount of water is from 0.01 to 90 wt%, preferably 0.01 to 50 wt%, more preferably from 5 to 45 wt%, even more preferably from 5 to 40 wt%, still more preferably from 5 to 30 wt%, wherein the weight percentages (wt%) are expressed with respect to the total weight of the fluid mixture of step a).

Preferably, the fluid mixture of step a) is in the form of an emulsion, microemulsion, nanoemulsion, suspension, solution, concentrated solution or heterogeneous mixture. Preferably, the fluid mixture of step a) is in the form of an emulsion, microemulsion, nanoemulsion, suspension, or solution.

The term "emulsion" refers to a fine dispersion of droplets of one liquid in another in which it is not soluble or miscible, preferably wherein the droplets have an average size of at least 1 mm. A "microemulsion" refers to an emulsion wherein the droplets have an average size in the range of 1 to less than 1000 $\mu$m. The term "nanoemulsion" refers to an emulsion wherein the droplets have an average in the range of 1 to less than 1000 nm. The term "average size" or "mean size", as used herein, relates to the average diameter of the droplets. The average size of these systems can be measured by standard processes known by persons skilled in the art such as dynamic light scattering.

The term "suspension" refers to a heterogenous mixture of one or more types of solid particles in a liquid without dissolving in it.

The term "solution" refers to a homogeneous mixture of substances, preferably in liquid state. A particular type of solution is a "concentrated solution", which refers to a solution as previously defined wherein the solute concentration is at least 70% of the saturation concentration, preferably is at least 80%, and more preferably is 99% of the saturation concentration of the specific solute.

The term "heterogeneous mixture" refers to a mixture comprising more than 2 phases, for example, two solid phases and a liquid phase, two liquid phases and a solid phase, each liquid phase comprising at least two components can be in the form of a solution, an emulsion, a microemulsion or a nanoemulsion.

The fluid mixture of step a) may be prepared by conventional methods known by the skilled person, such as mixture of the ingredients, dissolution of the ingredients, emulsification, homogenization at low or high pressure, dispersion, and/or heating to melt and mix solid components.

Preferably, the fluid mixture of step a) is provided at a temperature of from 4 °C to 250 °C, preferably of from 30 °C to 95°C, more preferably from 50 °C to 85 °C, still more preferably from 50 to 70 °C.

In particular, the fluid mixture of step a) is provided in the first vessel of the apparatus according to the second aspect of the invention.

The next step of the process of the invention, step b), is mixing the fluid mixture of step a) with a desiccating gas and/or plasticizing gas to provide a dispersion of the fluid mixture of step a) and the desiccating gas and/or plasticizing gas.

The term "dessicating gas" refers to any gas which is capable to remove water vapor from the sprayed moisture at lower temperatures than the boiling point. Examples of dessicating gases are nitrogen, dry air, helium, argon and mixtures thereof.

The term "plasticizing gas" refers to $CO_2$.

Preferably, the desiccating and/or plasticizing gas used in step b) is selected from the group consisting of nitrogen, $CO_2$, dry air, helium, argon and mixtures thereof. More preferably, the desiccating and/or plasticizing gas used in step b) is selected from the group consisting of nitrogen, $CO_2$ and mixtures thereof. In a preferred embodiment, the desiccating and/or plasticizing gas used in step b) is nitrogen. In another embodiment, the desiccating and/or plasticizing gas used in step b) is $CO_2$.

Preferably, the desiccating gas and/or plasticizing gas of step b) is provided at a pressure of from 0.2 to 16 bar, preferably from 1 to 5 bar, more preferably from 1 to 2 bar.

The desiccating and/or plasticizing gas flows are typically in a range between 0.3 and 0.7 g/s (0.8 and 2.0 m3/h respectively).

Preferably, step b) is carried out at a temperature of from 20 °C to 250 °C, preferably of from 30 °C to 95 °C and more preferably from 50 °C to 85 °C, still more preferably from 50 to 70 °C.

In particular, the desiccating and/or plasticizing gas of step b) is preheated (i.e. heated before mixing with the fluid mixture of step a)). Preferably, it is heated at a temperature similar to that of the fluid mixture of step a) so as to minimize the temperature gradient between the fluid mixture and the gas. Thus, preferably the desiccating and/or plasticizing gas is preheated at a temperature of from 20 °C to 250 °C, preferably of from 30 °C to 95 °C and more preferably from 50 °C to 85 °C, still more preferably from 50 to 70 °C.

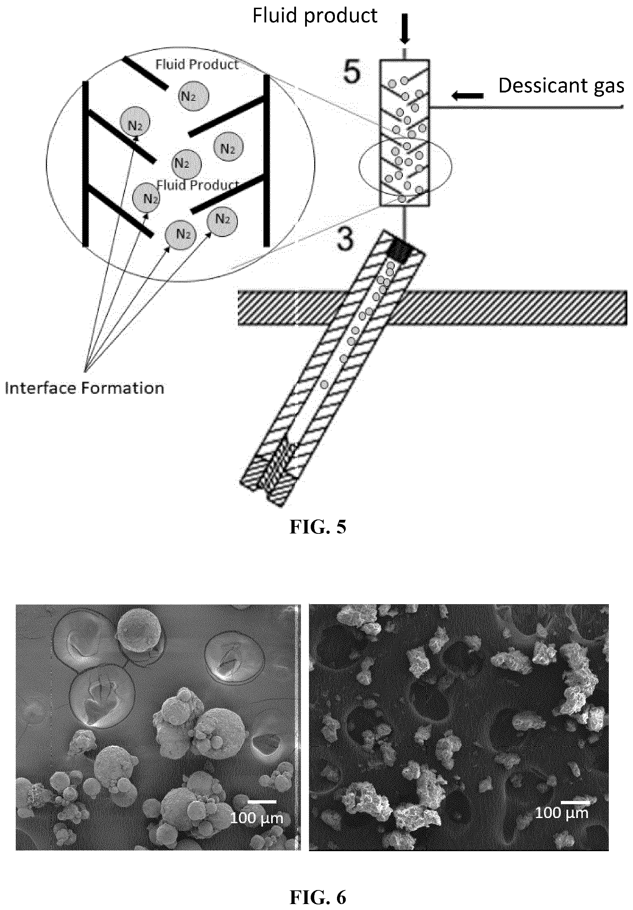

Step b) may be carried out in any device for mixing the fluid mixture provided in step a), with the desiccating gas and/or plasticizing gas which provides a dispersion of the fluid mixture and the desiccating gas and/or plasticizing gas. Examples of suitable devices for carrying out this mixing step are described for the apparatus according to the second aspect of the invention. Preferably, said device is a static mixer. A representation of the mixing step b) in a static mixer can be seen in Figure 5.

As known by the skilled person, a "dispersion" is a system in which distributed particles of one material are dispersed in a continuous phase of another material. In the context of the process of the invention, the particles refer to gas microbubbles (i.e. having a mean diameter within the range of 0.5 $\mu$m to less than 1 mm) and the continuous phase refers to the fluid mixture provided in step a).

The next step in the process of the invention, step c), is ejecting the dispersion obtained in step b) through a nozzle thereby creating a capillary or preatomized flow.

The term "capillary flow" is a flow under laminar regime wherein all molecules from the stream move in an orderly way. It refers to the laminar flow of the dispersion obtained in step b) in a narrow passageway such as a small internal diameter tube within the nozzle.

The term "preatomized flow" refers to a flow under a turbulent flow regime with chaotic movement of the molecules in the stream causing an aerosol-like flow (i.e. having microdroplets, in particular with a mean diameter below 1mm).

Any conventional nozzle may be used in step c). Examples of suitable nozzles for carrying are described for the apparatus according to the second aspect of the invention.

In particular, step c) is carried out at a temperature of from 20 °C to 250 °C, preferably of from 30 °C to 95 °C and more preferably from 45 °C to 85 °C.

The next step in the process, step d), is contacting the capillary or preatomized flow obtained in step c) with an expanding liquid or dense $CO_2$ swirling or spiraling flow to obtain the solid microparticles.

The term "liquid $CO_2$" refers to $CO_2$ having a pressure in the range of 5 bar to 70 bar and a temperature of -56 °C to 30 °C and which is in liquid state.

The term "dense $CO_2$" refers to $CO_2$ having a pressure in the range of 45 to 65 bar and a temperature of 20 °C to 30 °C.

The expression "expanding liquid or dense $CO_2$" refers to $CO_2$ which is provided as liquid or dense $CO_2$ and which progressively expands, i.e. it decreases its pressure to the range of atmospheric pressure (to 1-2 bar) until reaching a gaseous state.

Preferably, the expanding liquid or dense $CO_2$ of step d) is provided at a pressure before expansion of from 15 to 70 bar, preferably from 45 to 60 bar.

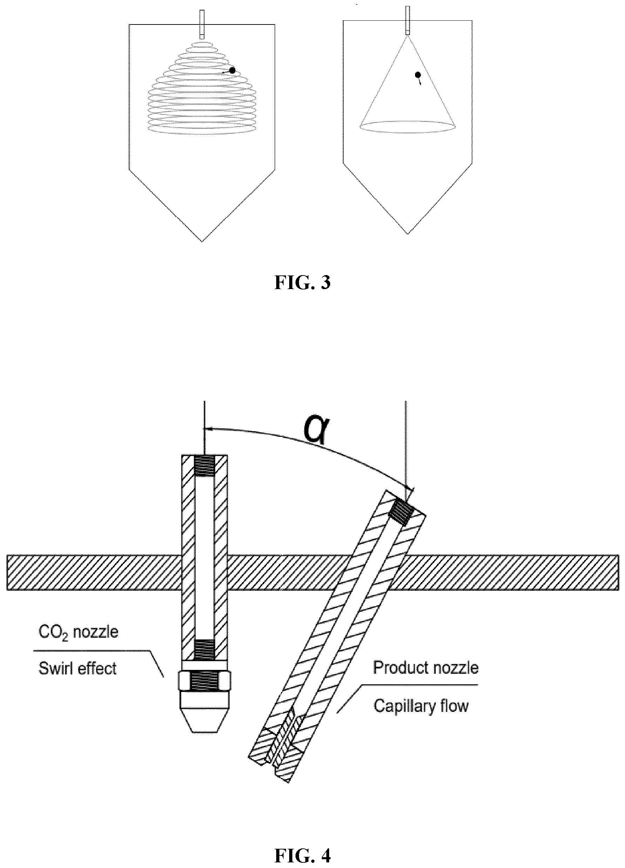

The term "swirling or spiraling flow" refers to a flow which moves with a twisting, whirling or rotating motion. A representation of a swirling flow in comparison to a conic flow can be seen in Figure 3.

Any nozzle suitable for ejecting a swirling or spiraling flow of a gas which is not in supercritical conditions may be used in step d). Examples of suitable nozzles are described for the apparatus according to the second aspect of the invention.

Process according to any one of the preceding claims, wherein the flows that are contacted in step d) (i.e. the capillary or preatomized flow obtained in step c) and the expanding liquid or dense $CO_2$ swirling or spiraling flow) are ejected at an angle of from 0° to 90° with respect to each other, preferably from 15° to 50°, more preferably from 30 to 45°.

The capillary or preatomized flow obtained in step c) may be ejected through one or more nozzles, whereas the liquid or dense $CO_2$ swirling or spiraling flow is generally ejected through a single nozzle.

The disposition of the nozzles of each flow is described below for the apparatus according to the second aspect of the invention.

When the capillary or preatomized flow obtained in step c) and the expanding liquid or dense $CO_2$ swirling or spiraling flow mix, the fluid product is rapidly cooled down below cryogenic temperatures and atomized due to the drastic pressure change caused by the expansion of the liquid $CO_2$ into gas, forming small, spherical microparticles (microspheronization) and also facilitates water removal, increasing the efficiency of drying, even at low temperatures reached during $CO_2$ expansion.

Flow rates can be regulated by flowmeter and/or by the type of nozzle, its diameter and the temperature conditions. Typically, flow rates of the capillary or preatomized flow obtained in step c) are between 1.0 and 12.0 g/s (4.0 - 42.0 kg/h). Typically, flow rates of liquid or dense $CO_2$ swirling or spiraling flow are between 1.5 and 75.0 g/s.

Examples of suitable vessels for carrying out d) step are described for the apparatus according to the second aspect of the invention. Preferably, said vessel is a spray tower.

Preferably, step d) is carried out at a temperature of from -20 °C to 35 °C.

The microparticles formed by the process of the invention may be collected from the bottom of the vessel of step d) or in a cyclone collector. Exhaust gases may abandon the vessel of step d) (e.g. spray tower) from the top and optionally pass through the filtering unit which collects the solid particles suspended and cleans the gases at the exit of the unit.

In a preferred embodiment, the fluid mixture of step a) is provided at a temperature of from 20 °C to 250 °C, preferably of from 30 °C to 95°C and more preferably from 50 °C to 85 °C step b) is carried out at a temperature of from 20 °C to 250 °C, preferably of from 30 °C to 95 °C and more preferably from 50 °C to 85 °C, step c) is carried out at a temperature of from 20 °C to 250 °C, preferably of from 30 °C to 95 °C and more preferably from 45 °C to 85 °C, and/or step d) is carried out at a temperature of from -20 °C to 35 °C.

The process of the present invention may be performed in batch or continuous.

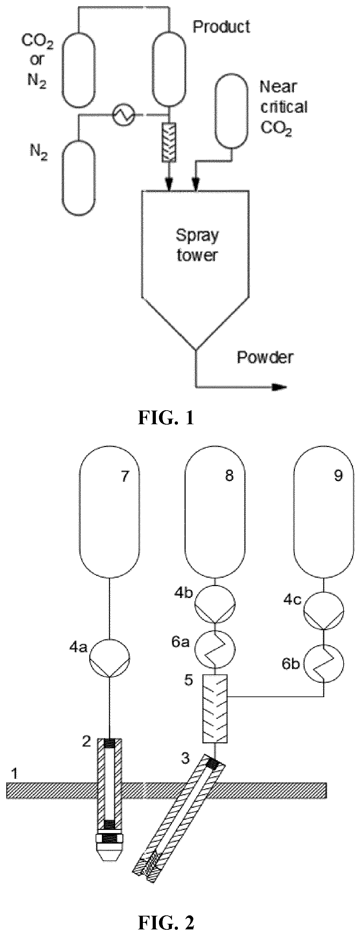

In a particular embodiment of the process of the invention, the spraying apparatus device of Figure 2 comprises a vessel where the product substance (i.e. the fluid mixture obtained in step a) of the process of the invention) is loaded and pressurized at low pressures with inert gas (9). The fluid product (i.e. the fluid mixture obtained in step a) of the process of the invention) is then dynamically mixed with a preheated dessicating gas flow (e.g. nitrogen) and/or a plasticizing gas flow (i.e. $CO_2$) in a static mixer (5), i.e. step b) of the process of the invention, and pushed through a temperature-controlled nozzle (3), i.e. step c) of the process of the invention, where it gets in contact with an expanding $CO_2$ flow coming from the liquid-dense $CO_2$ nozzle (2) connected to the liquid carbon dioxide container (7), i.e. step d) of the process of the invention. When the flows coming from nozzles (2) and (3) mix, the fluid product is rapidly cooled down below cryogenic temperatures and atomized due to the drastic pressure change caused by the expansion of the liquid $CO_2$ into gas. The fine microparticles formed by this dynamic interaction are collected from the bottom of the spray tower and the exhaust gases abandon the spray tower from the top and pass through the filtering unit which collects the powder suspended and cleans the gases at the exit of the unit. Finally, the dried, fine microparticles formed during the cryospraying operation are collected at the bottom of the spray tower or in a cyclone collector.

One of the main advantages of the process of invention is that it allows the production of small, spherical microparticles without the need of employing supercritical fluids.

Apparatus of the invention

In the second aspect, the present invention relates to an apparatus for performing the process according to the first aspect, the apparatus comprising:

i) a first vessel for providing the fluid mixture comprising the one or more molecules of interest, the one or more compounds selected from the group consisting of lipids, lipid-polymer compounds and polymers, and optionally water,

ii) a source of the desiccating gas and/or plasticizing gas,

iii) a device for mixing the fluid mixture comprising the one or more molecules of interest, the one or more compounds selected from the group consisting of lipids, lipid-polymer compounds and polymers and optionally water, with the desiccating gas and/or plasticizing gas to provide the dispersion of the fluid mixture and the desiccating gas and/or plasticizing gas,

iv) means for conveying the fluid mixture of the first vessel i) to the device iii),

v) means for conveying the desiccating gas and/or plasticizing gas from the source ii) to the device iii),

vi) a second vessel for microparticle formation,

vii) one or more nozzles for creating a capillary or preatomized flow of the dispersion of the fluid mixture and the desiccating gas and/or plasticizing gas located in the upper part of the third vessel vi),

viii) means for conveying the dispersion of the fluid mixture and the desiccating gas and/or plasticizing gas of the device iii) to the one or more nozzles vii),

ix) a source of the liquid or dense $CO_2$,

x) a nozzle for creating a swirling or spiraling flow of liquid or dense $CO_2$ located in the upper part of the second vessel vi),

xi) means for conveying the liquid or dense $CO_2$ from the source ix) to the nozzle x), and

wherein the one or more nozzles vii) are positioned at a distance with respect to nozzle x) of from 0.1 to 500 mm, preferably from 5 to 50 mm.

Item i) is the first vessel. This vessel is for providing the fluid mixture comprising the one or more molecules of interest, the one or more compounds selected from the group consisting of lipids, lipid-polymer compounds and polymers, and optionally water, which has been described in detail in the first aspect of the invention. Any conventional vessel used in the manufacture of pharmaceutical and cosmetic products may be used. These vessels are generally made of stainless steel AISI316L or superior grade. AISI304 can be used for alimentary production. The skilled person will determine the appropriate volume. In general, the volume of the first vessel is from 1 to 5 L starting from a length/diameter (L/D) relation between 7:1 and 1:1 depending on the size of the equipment. For industrial production, this vessel is upscaled up to 25 L or more, such as from 25 to 100 L. Preferably, the first vessel comprises heating means, so that the temperature conditions described in step a) of the process of the invention can be reached. In particular, the fluid mixture is loaded in the first vessel and pressurized at low pressures (e.g. below 10 bar, in particular from 1 to 4 bar) with inert gas (such as nitrogen, argon, helium or mixtures thereof, preferably nitrogen). In the present text, this vessel is also named as "product vessel". The first vessel i) is numbered as item 8 in Figure 2.

Item ii) is a source of the desiccating gas and/or plasticizing gas. The desiccating gas and/or plasticizing gas have been described in detail in the first aspect of the invention. Typically, the source of desiccating gas and/or plasticizing gas is a gas bottle or gas reservoir. Preferably having a volume of from 60 L to 5000 L. Generally, the desiccating gas and/or plasticizing gas is stored at a pressure of 45 to 300 bar and is provided in step b) of the process of the invention at a pressure of from 0.2 to 16 bar, preferably from 1 to 5 bar, more preferably from 1 to 2 bar. The source of the desiccating gas and/or plasticizing gas ii) is numbered as item 9 in Figure 2.

Item iii) is a device for mixing the fluid mixture comprising the one or more molecules of interest, the one or more compounds selected from the group consisting of lipids, lipid-polymer compounds and polymers and optionally water, with the desiccating gas and/or plasticizing gas to provide the dispersion of the fluid mixture and the desiccating gas and/or plasticizing gas. In this device, the fluid mixture is brought into contact with the desiccating gas and/or plasticizing gas generating a dispersion of the plasticizing and/or desiccating gas within the fluid mixture provided, generating a high interfacial area. Preferably said device is a static mixer. In Figure 2, the static mixer is numbered as item 5. Preferably, the device for mixing comprises heating means, so that the temperature conditions described in step b) of the process of the invention can be reached.

Item iv) is means for conveying the fluid mixture of the first vessel i) to the device iii). These means are typically a pipeline. These means may further include a valve. Preferably, means iv) comprise heating means, so that the temperature conditions described in step b) of the process of the invention can be reached. These heating means are identified as item 6a in Figure 2.

Item v) is means for conveying the desiccating gas and/or plasticizing gas from the source ii) to the device iii). These means are typically a gas pipeline. These means may further include a valve. Preferably, means iv) comprise heating means, so that the temperature conditions described in step b) of the process of the invention can be reached. These heating means are identified as item 6b in Figure 2.

Item vi) is a second vessel which is for microparticle formation. Any conventional vessel used in the manufacture of pharmaceutical and cosmetic products may be used. These vessels are generally made of stainless steel AISI 316L or superior grade. The skilled person will determine the appropriate volume. In general, the volume of the first vessel is from 1 to 5 L. Preferably, the second vessel is a spray tower.

Preferably, in the apparatus of the invention, device iii) is a static mixer and the second vessel vi) is a spray tower.

Item vii) is one or more nozzles for creating a capillary or preatomized flow of the dispersion of the fluid mixture and the desiccating gas and/or plasticizing gas. Conventional nozzles for creating a capillary or preatomized flow may be used. This represents an advantage with respect to prior art devices which need a complex and/or sophisticated nozzle design in order to obtain small, spherical and dry microparticles. Preferred nozzles can be heated by electrical resistance or can be heated by a circulating fluid (such as water, silicones, oils, etc) inside a jacket surrounding the nozzle. In figure 2, said nozzle is identified as item 3.

Said one or more nozzles vii) are located in the upper part of the second vessel vi). The term "upper part" refers to the upper two thirds of the second vessel vi), preferably to the upper half of the second vessel vi), more preferably to the upper third of the second vessel vi), more preferably to the top of the second vessel vi). The volume of the second vessel ranges from 20 L to 20,000 L, preferably from 60 L to 5000 L, and more preferably from 200 L to 2000 L. The length to diameter (L/D) relation of the second vessel ranges from 2:1 to 20:1, preferably between 3:1 to 1:6. The vessel is made of stainless steel AISI 316L or superior grade material suitable for pharma/food processing when necessary.

The term "one or more" includes, for example, 1, 2, 3, 4, 5, 6, 7, 8, 9 or 10. In one embodiment, one nozzle vii) is present. In another embodiment, more than one nozzle vii) is present, for example, 2, 3, 4, 5, 6, 7, 8, 9 or 10 nozzles.

Preferably, nozzles vii) comprise heating means, so that the temperature conditions described in step c) of the process of the invention can be reached.

Item viii) is means for conveying the dispersion of the fluid mixture and the desiccating gas and/or plasticizing gas of the device iii) to the one or more nozzles vii). These means are typically a pipeline made of stainless steel AISI 316L or superior grade. These means may further include a valve. Preferably, means viii) comprise heating means, so that the temperature conditions described in step c) of the process of the invention can be reached.

Item ix) is a source of the liquid or dense $CO_2$. The liquid or dense $CO_2$ has been described in detail in the first aspect of the invention. Typically, the source of liquid or dense $CO_2$ is a gas bottle, gas reservoir, gas tank, Dewar tank, etc. Preferably having a volume of from 60 to 5000 L. Generally, the liquid or dense $CO_2$ is stored at a pressure of 45 to 65 bar and is provided in step d) of the process of the invention at a pressure of from at a pressure of from 15 to 70 bar, preferably from 45 to 60 bar. In Figure 2, the source of the liquid or dense $CO_2$ is identified with number 7.

Item x) is a nozzle for creating a swirling or spiraling flow of liquid or dense $CO_2$. Conventional nozzles for creating a swirling or spiraling flow may be used. Examples of nozzles x) are nozzles with a swirling cavity inside the tip (swirling chamber) causing the spiral flow of the fluid stream and made of AISI 316L or superior grade. In Figure 2, nozzle x) is identified with number 2.

Nozzle x) is located in the upper part of the second vessel vi). As explained above, the term "upper part" refers to the upper two thirds of the second vessel vi), preferably to the upper half of the second vessel vi), more preferably to the upper third of the second vessel vi), more preferably to the top of the second vessel vi).

In step c) of the process of the invention, the capillary or preatomized flow obtained in step c) is contacted with the expanding liquid or dense $CO_2$ swirling or spiraling flow. For this contact to occur, each of the one or more nozzles vii) are positioned at a distance with respect to nozzle x) of from 0.1 to 500 mm, preferably from 5 to 500 mm, more preferably from 5 to 50 mm. Said "distance" is determined as the distance from the tip of nozzle(s) vii) to the tip of nozzle x).

In particular, when the apparatus comprises more than one nozzle vii), each of said nozzles vii) is positioned at the same distance to nozzle x).

In a preferred embodiment, in the apparatus of the invention, the one or more nozzles vii) are at an angle of from 0° to 90° with respect to the nozzle x), preferably from 15° to 50°, more preferably from 30° to 45°. This angle is identified as $\alpha$ in Figure 4. An example of two nozzles at an angle of 0° is both nozzles projecting their corresponding flow vertically downwards.

The angle is determined as the angle formed by the flows projected from each nozzle, as shown in Figure 4.

In particular, when the apparatus comprises more than one nozzle vii), each of said nozzles vii) is at the same angle with respect to nozzle x).

In a preferred embodiment, the one or more nozzles vii) are positioned with respect to nozzle x) to provide a distance of incidence from 0 mm up to 300 mm, preferably between 5 and 50 mm and more preferably between 20 and 30 mm. The "distance of incidence" is determined as the distance from the tip of nozzle(s) vii) to the point at which the flow from nozzle(s) vii) and the flow from nozzle x) meet.

In particular, when the apparatus comprises more than one nozzle vii), each of said nozzles vii) is positioned with respect to nozzle x) to provide the same distance of incidence.

In a preferred embodiment, in the apparatus of the invention, nozzle x) is positioned vertically, i.e. with the tip of the nozzle facing downwards, as shown in Figures 2 and 4.

In the apparatus according to the invention, the nozzles may be disposed on an interchangeable plate to allow the easy disassembling of one plate from the top of the second vessel (e.g. spray tower), and the mounting of other plates with different types of nozzles, or angle of incidence. The plate is identified with number 1 in Figure 2.

The nozzles plate can be designed to hold multiple nozzles vii) placed around a central nozzle x). Nozzles can be placed at different angles on a horizontal plane, while maintaining the same incidence angle to the central $CO_2$ nozzle. In a typical manner, two product nozzles can be placed at 180° one from the other, three nozzle at 120° or four nozzles at 90° one from the other. Such arrangement can increase the productivity.

The flow rate of each of the one or more nozzles vii) is in the range from 4.0 to 170 kg/h, preferably from 4.0 to 126 kg/h, more preferably from 4.0 to 84.2 kg/h, still more preferably from 4.0 to 42.0 kg/h. The typical flow rate when there is one nozzle vii) is in a range between 4.0 and 42.0 kg/h. With the arrangements to increase productivity product mass flow can reach rates up to 84.2 kg/h in the case of two product nozzles, 126.0 kg/h in the case of three product nozzles and 170 kg/h in the case of four product nozzles; these mass flows refer to the combined flow rates from all nozzles vii), i.e. the sum of the mass flows from all nozzles vii).

Another embodiment of the invention allows the inclusion of multiple nozzle plates each plate featuring one nozzle x) in central position and one or more nozzles vii) placed at an adequate angle of incidence. In such manner, it is possible to assemble a large spray tower with multiple spraying plates in a relatively small space.

Item xi) is means for conveying the liquid or dense $CO_2$ from the source ix) to the one or more nozzles x). These means are typically a gas pipeline made of AISI 316L or superior grade. This pipeline usually comprises a thermal insulator from the $CO_2$ source to nozzle x) in the apparatus, to avoid temperature changes during flow, to avoid density change and risks of transition from liquid to gas $CO_2$ before the nozzle x).

Preferably, in the apparatus of the invention, the first vessel i), the device ii), the means iv), the means viii), the one or more nozzles vii) comprise heating means. Examples of heating means are heat exchangers (shell and tubes or double tube operating at counter or direct current) consisting on circulating heating baths or any mean to provide a flow of hot fluid. Additionally, electrical resistances in form of heating belts or wires can be installed.

Flow rates can be regulated by flowmeter and/or by the type of nozzle, its diameter and the temperature conditions of each flow (i.e. the dispersion obtained in step b) of the process of the invention, which comprises the molecule of interest and the expanding liquid or dense $CO_2$). Typically flow rates of the dispersion obtained in step b) are between 1.0 and 12.0 g/s (between 4.0 and 42.0 kg/h). The flow rates of liquid or dense $CO_2$ are usually between 1.0 and 75.0 g/s, preferably 1.5 and 24 g/s. The desiccating and/or plasticizing gas flows are typically in a range between 0.3 and 0.7 g/s (between 0.8 and 2.0 m$^3$/h).

A general representation of an apparatus according to the invention is provided in Figures 1 and 2.

The following examples represent specific embodiments of the present invention. They do not intend to limit in any way the scope of the invention defined in the present description.

## EXAMPLES

### Materials and methods

**Apparatus.** A general representation of an apparatus according to the invention is provided in Figures 1 and 2, and described in detail in the previous section. In the examples described below the apparatus was set using a first vessel of 1 Liter volume capacity, a mixing device (static mixer), and a spray tower of 60 L volume capacity. The angle between the product fluid nozzle and the $CO_2$ set at $\alpha$ = 30°, and the nozzles distance set at 25 mm, unless indicated otherwise in the specific examples. Process conditions are specifically indicated in each example.

**Water elimination.** The residual water content in the microparticles was measured by water titration (Karll-Fisher method) on the recovered powders. The results are normalized calculating the WRE (Water Removal Efficiency) according to the relation:

$$\%Efficiency = \frac{\%H_2O_i - \%H_2O_f}{\%H_2O_i} \cdot 100$$

where $H_2O_i$ is the water content in the formulation before spraying, and $H_2O_f$ is the residual water in the powder microparticles.

**Particle morphology.** Particle morphology was assessed by scanning electron microscopy (SEM) and by optical microscopy. JEOL JSM-6010L Scanning Electron Microscope (SEM) was used to examine microparticles size and morphology. Samples in powder form were coated with conductive material (gold) and 3D image was obtained from the flux of electrons passing through the sample.

An optical microscope Olympus BH-2 was used to observe the micronized powders after processing. Dry microparticles samples were placed on a glass slide and gently covered with a glass slip, without applying pressure, and observed at different magnification. Photographs of selected fields and magnifications were taken.

**Particle size distribution.** Particle size distribution was analyzed using a Malvern MasterSizer 3000. particles analyzer equipment (Malvern). To perform the analyses, a small amount of the micronized powders was suspended in 0.1 to 0.5 % Tween 80 aqueous solution to facilitate the separation of potential aggregates before the analysis.

Furthermore, microspheres described in Examples 27 (sample DE042, Table 12), (sample DE054, Table10) and Example 28 (samples SMX003 and SMX 004) were also analyzed for particles size distribution with a Malvern Morphologi 4 analyzer, applying a dry dispersion protocol that allows to directly analyze the samples in powder form. In this analysis, the instrument achieves a physical separation of particles in dry powder form, and captures images of individual particles by scanning the sample underneath the microscope high resolution optics. The same Morphologi4 analyzer directly calculated particles circularity, by measuring the Area A and the perimeter P of microparticles images in each sample

**Circularity,** C (dimensionless value), is defined as the degree to which the visualized particle is similar to a circle, taking into consideration the smoothness of the perimeter. Therefore, circularity is a measurement of both the particle form and its roughness. Thus, the closest to a perfectly round, smooth circle a particle is, the closest the circularity value is to 1.0. Circularity is defined as

$$C = \sqrt{\frac{4\pi A}{P^2}}$$

where A is the area of the observed particle, and P is its perimeter. A and P are determined using the particle morphology assay described above.

**Comparative Example *1. Microparticles production and water elimination capacity using a swirling flow nozzle for CO_2 expansion, in combination with capillary flow product nozzle***

A formulation composed of water-soluble and water-dispersible excipients, water and a hydrosoluble and hygroscopic active molecule (clindamycin phosphate, API) was prepared by dissolving the API in a blend of the water-miscible excipients, and subsequently emulsifying the blend with the water-dispersible excipients, maintained at a temperature slightly higher than their melting temperature, generally between 50° and 60° C. Two emulsion formulations, their composition described in the following Table 1, were prepared and used for the experimental sets 1 and 2. Formulations described in experimental set 1 and 2 differ for the amount of water contained in the emulsion.

Table 1. Biphasic formulations sprayed according to the Example 1.

| Experimental SET Formulation | Geleol (%) | Glycerin (%) | Transcutol (%) | Water (%) | Clindamycin phosphate (%) |
|---|---|---|---|---|---|
| 1 | 50.0 | 16.7 | 16.7 | 6.6 | 10.0 |
| 2 | 44.0 | 9.4 | 18.6 | 18.6 | 9.4 |

The same processing conditions were applied to experimental Sets 1 and 2. Each formulation was poured into the product vessel of the apparatus of the invention described in Figure 1 and Figure 2, equilibrated at controlled temperature and pressure, and sprayed. The following processing conditions were applied: Product vessel temperature:

65.0 °C; Product line temperature: 70.0°C; Product nozzle temperature: 85.0 °C; $N_2$ pressure applied to product: 3.5 bar; Liquid $CO_2$ pressure: 56 bar.

The product nozzle configuration was set for the generation of a capillary flow. The $CO_2$ nozzle configuration was set to generate the swirling/spiralling flow pattern of expanding $CO_2$, as described in the invention. After opening the $CO_2$ valve to generate the swirling flow, the product valve was opened to let the product flow entering into contact with the expanding $CO_2$. At the end of the procedure, valves were closed, and the micronized powders were recovered at the bottom of the spray-tower and characterized for residual water content (Karl-Fisher Analysis), particles size, particles morphology and DSC.

**Comparative Example 2. *Microparticles production and water elimination capacity using a hollow cone flow nozzle for $CO_2$ expansion, in combination with capillary flow product nozzle***

The same formulations described in Table 1 were prepared and each of them sprayed with a cryospraying equipment in the configuration described in prior art EP2298286B 1. The configuration according to prior art differs from that used in Comparative Example 1 in the use of a $CO_2$ nozzle (0.5 mm diameter) producing a hollow-cone flow pattern, instead of the $CO_2$ nozzle (0.5 mm diameter) producing the swirling flow pattern. All other processing conditions were applied as described in Comparative Example 1. Each formulation was poured into the product vessel of the apparatus configured as per prior art, equilibrated at controlled temperature and pressure, and sprayed. Conditions applied: Product vessel temperature: 65.0 °C; Product line temperature: 70.0°C; Product nozzle temperature: 85.0 °C; $N_2$ pressure applied to product: 3.5 bar; Liquid $CO_2$ pressure: 56 bar. Microparticles produced were collected and characterized for the same parameters described for Comparative Example 1.

**Results of comparative examples 1 and 2: analysis of water content and morphology of the microparticles**

The results reported in Table 2 show the advantage of the use of a swirling flow nozzle for $CO_2$ expansion in eliminating water during the spraying of a hygroscopic formulation under $CO_2$ expansion.

Table 2. Effect of $CO_2$ flow pattern on water elimination.

| Example | Set | Flow pattern of $CO_2$ | $H_2Oi$ (%) | $H_2Of$ (%) | Water removal efficiency (%) |
|---|---|---|---|---|---|
| **Comparative example 1** | Set 1 | Swirl flow | 6.8 | 5.3 | 22.5 |
| | Set 2 | Swirl flow | 18.9 | 11.2 | 40.7 |
| | Set 2 | Swirl flow | 18.9 | 12.7 | 32.8 |
| | Set 2 | Swirl flow | 18.9 | 10.6 | 43.9 |
| | Set 2 | Swirl flow | 16.4 | 7.8 | 52.4 |
| | Set 2 | Swirl flow | 16.4 | 10.3 | 37.2 |
| Comparative example 2 | Set 1 | Hollow Cone | 6.7 | 9.0 | -34.3 |
| | Set 1 | Hollow Cone | 6.7 | 8.0 | -19.4 |
| | Set 2 | Hollow Cone | 18.6 | 13.9 | 25.3 |
| | Set 2 | Hollow Cone | 18.6 | 16.0 | 14.1 |

The results show that water removal efficiency is always higher when the product is sprayed using the swirling flow $CO_2$ nozzle (Comparative Example 1), than using the hollow cone $CO_2$ flow nozzle. These results are of particular importance since some of the excipients used and the API itself are quite hygroscopic and have strong affinity for water, therefore they can bind and retain it.

Particle morphology of the microparticles obtained in Comparative Examples 1 and 2 was compared. Scanning electron microscopy (SEM) images obtained on microspheres produced according to Comparative Example 1 (spherical microparticles) and Comparative Example 2 (fragmented microparticles of different shapes) are shown in Figure 6. It is clearly evident how the spiraling pattern of expanding $CO_2$ facilitates the formation of perfectly spherical particles and having a controlled size distribution when compared to the particles obtained using a hollow cone $CO_2$ flow.

**Example 4. *Microparticles production using a swirling flow nozzle for $CO_2$ expansion, in combination with mixing of the sprayable substance with a gas in a static mixer (preatomization)***

The equipment configuration was set for the generation of a preatomized flow, with the use of a gas-fluid static mixer in the product line. The $CO_2$ nozzle configuration was set to generate the spiraling flow pattern of expanding $CO_2$.

After opening the $CO_2$ valve to generate the swirling flow, the product valve was opened to let the nitrogen-product mixture entering into contact with the expanding $CO_2$. The experiment was performed using a composition containing 99.5% of a well-known and characterized excipient, glyceril-tristearate (GTS), and 0.5% of curcumin natural extract.

The following processing conditions were applied: Product vessel temperature: 65.0 °C; Product line temperature: 70.0 °C; Product nozzle temperature: 85.0 °C; $N_2$ pressure applied to product: 3.5 bar; Liquid $CO_2$ pressure: 56 bar. $CO_2$ nozzle diameter (0.5mm). Product nozzle diameter (0.5 mm). At the end of the procedure, valves were closed, and the micronized powders were recovered at the bottom of the spray-tower and characterized. Two different lots were prepared.

**Comparative Example *5. Microparticles production using a swirling flow nozzle for CO_2 expansion, without preatomization static mixer***

The following example describes the preparation of microparticles as described in Example 4 but differing from said example 4 only in that the equipment configuration without preatomization by static mixing is used. All the other conditions are the ones reported for Example 4.

**Results of Example 4 and Comparative Example 5**

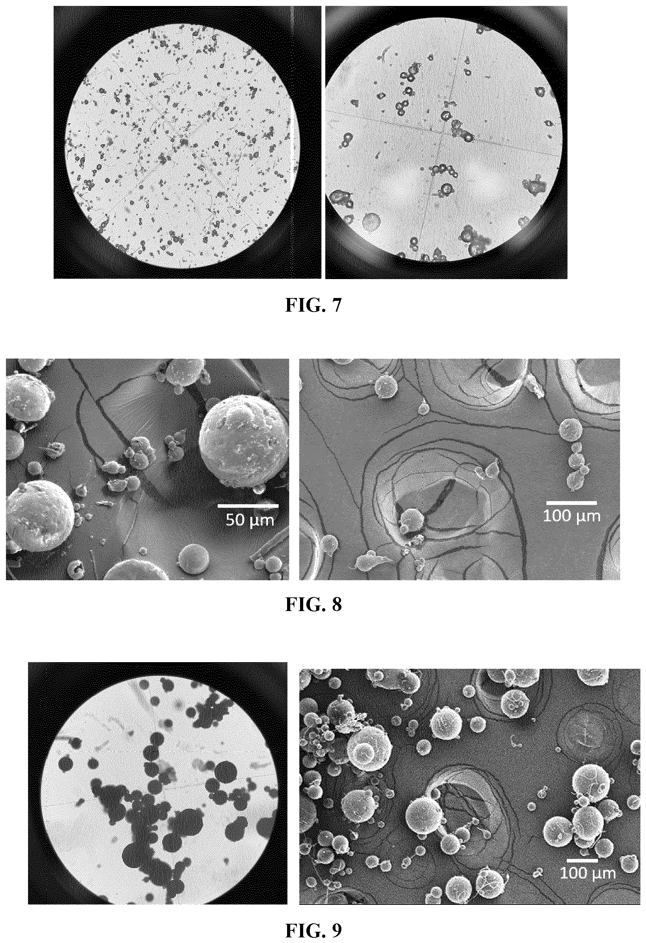

Microparticles produced in Example 4 and Comparative Example 5 were characterized with optical and electronical microscopy. Particle size distribution was also determined. As it can be seen in Figures 7 and 8 when compared to Figure 9, the spraying of the preatomized, expanding product into the swirling/spiralling $CO_2$ flow with a preatomization in a static mixer allows to produce spherical microspheres with smaller size.

Particles size distribution (in µm) of particles produced according to Example 4 and Comparative Example 5 is reported in the following Table 3, confirmed the positive effect of double cryogenic expansion on size distribution

Table 3. Cumulative distribution (by volume) of particles size of samples produced with and without static mixer configuration (Example 4 and Comparative Example 5, respectively)

| Sample | Dx (10) | Dx (50) | Dx (90) |
|---|---|---|---|
| Example 4 Lot SMX003 | 5.5 | 45.1 | 103 |
| Example 4 Lot SMX 004 | 6.2 | 30.9 | 94 |
| Comparative Example 5 Lot P32 | 38.6 | 93.3 | 195 |

Particles obtained according to the invention (Example 4), with static mixer equipment configuration combined with swirling flow $CO_2$ nozzle, are significantly smaller and with narrower particles size distribution. Microparticles with spherical morphology and with bigger specific surface area offer important advantages in terms of flowability, mass diffusion and dispersion or solubilization in liquid media.

**Comparative Example 6. *Microparticles production and water elimination capacity using a swirling CO_2 flow nozzle in combination with capillary product flow***

In this study we perform a cross-comparison of equipment configurations by processing the same formulations so to assess the effect of the swirling $CO_2$ flow without the use of static mixer in the product line (comparative example 6) and compare it with the effect of the static mixer configuration in the product line in combination with a hollow cone $CO_2$ flow (comparative example 7). An emulsifying agent (lipid-polyoxyl esthers, Gelucire 50/13) with capacity to coordinate and retain water was blended with a structuring lipid (glyceryl-di-behenate, Compritol 888), in a 15:85 ratio, and emulsified with amounts of water ranging from 5 to 40% w/w. The emulsions were sprayed at the following conditions: Product vessel temperature: 80.0 °C; Product line temperature: 80.0 °C; Product nozzle temperature: 85.0 °C; Product nozzle diameter: 0.85 mm; Product pressure($N_2$): 3.5 bar; Pressure $CO_2$: 55 bar; $CO_2$ nozzle diameter (0.50 mm - spiraling flow).

**Comparative Example 7. *Microparticles production and water elimination. Use of static mixer in product line in combination with hollow cone CO_2 flow***

The same formulations of comparative example 6, comprising an emulsifying agent (lipid-polyoxyl esthers, Gelucire 50/13) with a structuring lipid (glyceryl-di-behenate, Compritol 888), in a 15:85 ratio, was sprayed at the same conditions described in Comparative Example 6, except that this configuration comprises the static mixer in the product line, operating at 1,5 bar pressure, and a 0.5 mm nozzle with hollow-cone $CO_2$ flow, instead of the spiraling flow $CO_2$ nozzle described in comparative example 6. Product pressure($N_2$): 2 bar.

Results comparison from Comparative Examples 6 and 7 is provided in Table 4.

Table 4. Operation parameters, main variables and effect of $CO_2$ flow pattern on water removal

| Example | Sample | PN2 product line (bar) | Static Mixer | $CO_2$ Flow pattern | %$H_2O$i (contained in emulsion) | %$H_2O$f Residual in particles | Water removal efficiency (%) |
|---|---|---|---|---|---|---|---|
| 6 | DE007 | 3.5 | No | Swirl | 10.0 | 2.2 | 78.00 |
| | DE008 | 3.5 | No | Swirl | 20.0 | 10.0 | 50.00 |
| | DE009 | 3.5 | No | Swirl | 30.0 | 16.0 | 46.67 |
| 7 | DE039 | 2.0 | Yes | Hollow | 10.0 | 5.4 | 46.00 |
| | DE040 | 2.0 | Yes | Hollow | 20.0 | 11.3 | 43.50 |
| | DE041 | 2.0 | yes | Hollow | 30.0 | 18.3 | 39.00 |

Water removal efficiency is higher when samples are processed according to comparative example 6 (samples DE 007,008,009), with the equipment configured with the swirling $CO_2$ flow nozzle. Efficiency in water removal is limited when hollow cone $CO_2$ nozzle is used, even if in combination with the static mixer-preatomization in the product line (Comparative Example 7, samples DE 0039,040,041).

### Example 8. *Effect on different formulation compositions in microparticle production and water elimination capacity*

Formulations containing an emulsifying agent (lipid-polyoxyl esters, Gelucire 50/13) combined with a structuring lipid (glyceryl-di-behenate, Compritol 888), were sprayed applying the following processing conditions: Product vessel temperature: 80.0 °C; Product line temperature: 80.0 °C; Product nozzle temperature: 85.0 °C; Product nozzle diameter: 0.85 mm; Product pressure ($N_2$): 3.5 bar; Pressure $CO_2$: 55 bar; $CO_2$ nozzle diameter (0.50 mm - spiraling flow). Mixtures at different ratios (R) of lipid-polyoxyl esters (Gelucire 50/13) and glyceril di-behenate (Compritol 888) were used (R= 10:90; 15:85; 30:70; Gelucire:Compritol). Each composition at the different lipid-polyoxyl esters/glyceril di-behenate fixed ratio R was prepared and emulsified with water, so that the initial water content in the formulations was 10%, 20%, 30% and 40% by weight, and consequently the content of the lipid-polyoxyl esters / glyceril di-behenate mixture in the composition was 90%, 80%; 70% and 60% respectively. Herebelow, we report the % composition of all the mixtures prepared.

| Gelucire / Compritol ratio R | Gelucire 50/13 (%w/w) | Compritol 888 (%w/w) | $H_2O$i (% w/w) |
|---|---|---|---|
| **10:90** | 9 | 81 | 10 |
| | 8 | 72 | 20 |
| | 7 | 63 | 30 |
| | 6 | 54 | 40 |
| **15:85** | 13.5 | 76.5 | 10 |
| | 12 | 68 | 20 |
| | 10.5 | 59.5 | 30 |
| | 9 | 51 | 40 |
| **30:70** | 27 | 63 | 10 |
| | 24 | 56 | 20 |
| | 21 | 49 | 30 |
| | 18 | 42 | 40 |

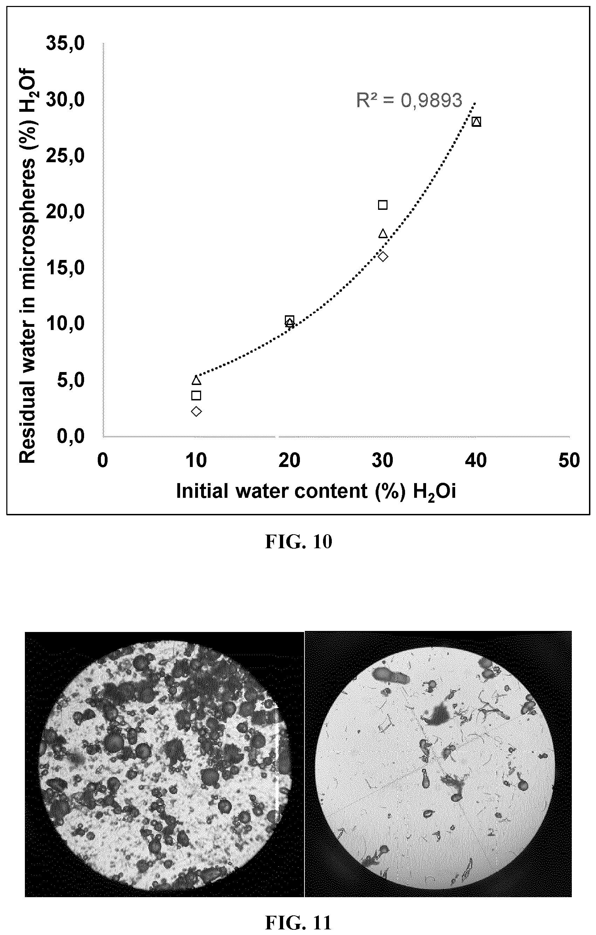

Each described formulation was then sprayed at the processing conditions mentioned. After spraying, microspheres could be recovered in all cases, and water residual content was determined. The residual water content in the powders obtained ($H_2O$f) is plotted in Figure 10 as a function of the amount of water initially contained in the emulsion sprayed ($H_2O$i). In all samples studied it was possible to obtain solid microparticles with a reduced amount of residual water in the powder, even at 40% initial water content and above. It can be noticed that the water removal efficiency by the use of swirling flow $CO_2$ nozzle does not depend from the formulation composition, since the differences observed for the three composition are minimal for each x-axis point.

**Example 9.** *Microparticles production and water elimination capacity according to the invention*

Formulations containing Gelucire 50/13:Compritol 888 (10:90 ratio) were prepared as described in Example 8. Spraying process operational conditions are: product vessel temperature: 80.0 °C, product line temperature: 80.0 °C, product nozzle temperature: 85.0 °C, product nozzle diameter: 0.85 mm, product pressure ($N_2$): 3.5 bar, Pressure $CO_2$: 55 bar, $CO_2$ nozzle diameter (0.5 mm - spiraling flow nozzle). In this experimental set, three emulsion formulations with a fixed ratio of Gelucire/Compritol and different amount of water were sprayed TEST DE 030,031,032).

**Comparative Example 10.** *Microparticles production and water elimination capacity using a $CO_2$ swirling flow pattern without static mixer*

For comparison with Example 9, the same formulations of Example 9 were processed using the same conditions as described in Example 9 but without the static mixer (TEST DE 017, 018, 019). All microspheres were produced, recovered as solid powder, and analyzed for water content by Karll Fisher.
**Results of example 9 and comparative example 10.** The results of Example 9 and Comparative Example 10 are reported in Table 5.

Table 5. Effect of static mixer in combination with $CO_2$ swirl nozzle on water removal from microparticles (cold-drying).

| Ex. | TEST ID | P $N_2$ (bar) | P$N_2$ mixer (bar) | $CO_2$ flow pattern | %$H_2$Oi | %$H_2$Of | Water removal efficiency (%) |
|-----|---------|---------|---------|------------|----------|----------|------------------|
| 9 | DE030 | 3.5 | 3.0 | Swirl flow | 10.0 | 1.1 | 89.00 |
| | DE031 | 3.5 | 3.0 | Swirl flow | 20.0 | 4.9 | 75.50 |
| | DE032 | 3.5 | 3.0 | Swirl flow | 30.0 | 16.3 | 45.67 |
| | DE017 | 3.5 | no | Swirl flow | 10.0 | 3.6 | 64.00 |
| 10 | DE018 | 3.5 | no | Swirl flow | 20.0 | 10.3 | 48.50 |
| | DE019 | 3.5 | no | Swirl flow | 30.0 | 20.6 | 31.33 |

As it can be seen, there is a clear advantage in water removal efficiency when using the process of the invention (Example 9).

**Example 11.** *Microparticles production and water elimination capacity according to the invention*

Formulations containing Gelucire 50/13:Compritol 888 (15:85 ratio) were prepared as described in Example 8. Example 11 differs from example 9 only for the use of modified formulations, containing a higher amount of the water-binding excipient (Gelucire 50/13), therefore representing a harder challenge to the process in achieving water removal. The same processing conditions applied in Example 9 were used here. In the experimental set, three emulsion formulations with increasing amount of water were sprayed with the equipment configured for the double cryogenic expansion (static mixer + spiraling flow $CO_2$ nozzle; TEST DE 042,043,044). Microspheres were produced, recovered and analyzed for water content by Karl Fisher.

**Comparative Example 12.** *Microparticles production and water elimination capacity with $CO_2$ swirling flow pattern without static mixer*

For comparison with Example 11, the same formulations were processed without the static mixer (capillary nozzle + spiralling flow $CO_2$; TEST DE 007,008,009). Microspheres were produced, recovered and analyzed for water content by Karl Fisher.
**Results of example 11 and comparative example 12.** The results of Example 11 and Comparative Example 12 are reported in Table 6.

Table 6. Effect of static mixer in combination with $CO_2$ swirl nozzle on water removal from microparticles (cold-drying).

| Example | TEST | PN$_2$ (bar) | PN$_2$ Mixer (bar) | CO$_2$ flow pattern | %H$_2$Oi | %H$_2$Of | Water removal efficiency (%) |
|---|---|---|---|---|---|---|---|
| 11 | DE042 | 2.0 | 1.5 | Swirl flow | 10 | 0.9 | 91.4 |
| | DE043 | 2.0 | 1.5 | Swirl flow | 20 | 8.2 | 59.0 |
| | DE044 | 2.0 | 1.5 | Swirl flow | 30 | 14.0 | 53.0 |
| 12 | DE007 | 3.5 | NO | Swirl flow | 10 | 2.2 | 78.0 |
| | DE008 | 3.5 | NO | Swirl flow | 20 | 10.0 | 50.0 |
| | DE009 | 3.5 | NO | Swirl flow | 30 | 16.0 | 46.7 |

The advantage of the double cryogenic expansion , i.e. combined use of preatomization by static mixer and swirling flow $CO_2$ pattern on water removal during $CO_2$-cryospraying is clearly evident also in this case, even in the presence of higher amounts of hygroscopic excipients in the microspheres formulation. In all cases the water removal efficiency resulted superior when the swirl-flow $CO_2$ nozzle is used in combination with the static mixer for preatomization.

**Comparative Example 13. *Microparticles production and water elimination capacity combining preatomization (static mixer in product line) with $CO_2$ cone pattern* and**

**Example 14. *Microparticles production and water elimination capacity according to the invention combining preatomization (static mixer in product line) with $CO_2$ swirling flow pattern)***

In the Examples 13 and 14, we compare the effect of the $CO_2$ flow pattern on water removal efficiency, when the static mixer configuration (preatomization) is applied to product spraying, performing the double cryogenic expansion in two different conditions of $CO_2$ flow. Experimental tests DE039-DE041 were performed on the Gelucire 50/13: Compritol 888 15:85 ratio formulation described in Example 11, using the cone $CO_2$ flow nozzle. Experiments DE042-DE044 were performed on the same formulation using the swirling $CO_2$ flow nozzle. Microspheres in powder form were collected and residual water content was analyzed by Karl-Fischer (see Table 7). Process equipment configuration and applied operational conditions are the following: product vessel temperature: 80.0 °C, line temperature: 80.0 °C; Product nozzle temperature: 85.0 °C; Product nozzle diameter: 0.85 mm; Product pressure (N$_2$): 2.0 bar; Pressure $CO_2$: 55 bar; $CO_2$ nozzle diameter (0.5 mm - spiraling flow nozzle; 0.5 mm hollow cone flow nozzle).

Table 7. Effect of different $CO_2$ flow patterns on water removal efficiency when product is preatomized with static mixer spraying apparatus.

| Examples | TEST | PN$_2$ (bar) | CO$_2$ flow pattern | %H$_2$Oi | %H$_2$Of | Water removal efficiency (%) |
|---|---|---|---|---|---|---|
| 13 | DE039 | 2.0 | Hollow Cone | 10.0 | 5.4 | 46.00 |
| | DE040 | 2.0 | Hollow cone | 20.0 | 11.3 | 43.50 |
| | DE041 | 2.0 | Hollow cone | 30.0 | 18.3 | 39.00 |
| 14 | DE042 | 2.0 | Swirl | 10.0 | 0.9 | 91.35 |
| | DE043 | 2.0 | Swirl | 20.0 | 8.2 | 59.00 |
| | DE044 | 2.0 | Swirl | 30.0 | 14.0 | 53.00 |

The results reported in Table 7 indicate that unexpectedly, the efficacy of $CO_2$-based double cryogenic expansion in water removal (cold-drying) is much more effective if the preatomization by the use of a static mixer is performed in combination with a spiralling flow pattern of expanding, liquid $CO_2$. By using the swirl flow nozzle for $CO_2$, a much higher water removal efficiency can be obtained. It is also evident that the combination of preatomization by static mixer with the swirling-flow $CO_2$ nozzle is more effective in obtaining dryer microspheres independently of the initial water content of the sprayed product.

**Comparative Example *15. Microparticles production and water elimination capacity using swirling* $CO_2$ *nozzle in combination with capillary flow product nozzle* and**

**Comparative Example 16.** *Microparticles production and water elimination capacity using a cone flow* $CO_2$ *nozzle in combination with capillary flow product nozzle*

In these examples we compare the water removal efficiency by the use of swirl $CO_2$ flow nozzle vs. the use of cone $CO_2$ nozzle in the cryospraying process. In both cases, the static mixer line was not used, so the product nozzle was set to generate a capillary flow. The same formulation as described in Example 11 (Gelucire 50/13- Compritol 888 in a 15:85 ratio) was used in these sets of experiments. Process conditions were: vessel temperature: 80.0°C; Product line temperature: 80.0 °C; Product nozzle temperature: 85.0 °C; Product nozzle diameter: 0.85 mm. Product pressure ($N_2$): 3.5 bar; Pressure $CO_2$: 55 bar. Example 15: $CO_2$ nozzle diameter: 0.5 mm spiraling flow nozzle. Example 16: 0.5 mm cone flow nozzle. The results are reported in Table 8

Table 8. Effect of use of swirl or cone - $CO_2$ nozzles on water removal efficiency, on apparatus configured without static mixer.

| Example | TEST | $PN_2$ (bar) | $CO_2$ flow | %H2Oi | %H2Of | Water removal efficiency (%) |
|---|---|---|---|---|---|---|
| | DE007 | 3.5 | Swirl | 10 | 2.2 | 78.0 |
| 15 | DE008 | 3.5 | Swirl | 20 | 10.0 | 50.0 |
| | DE009 | 3.5 | Swirl | 30 | 16.0 | 46.7 |
| | DE052 | 3.5 | Cone | 10 | 4.5 | 55.0 |
| 16 | DE053 | 3.5 | Cone | 20 | 11.8 | 41.0 |
| | DE054 | 3.5 | Cone | 30 | 17.2 | 42.7 |

The results indicate that the swirling flow of $CO_2$ offers an important technical advantage in terms of water removal efficiency during spraying at same experimental conditions.

Furthermore, by directly comparing the results of Example 14 with the results of Comparative Example 15, the unexpected advantage of combining the use of the static mixer (preatomization) configuration with spiralling flow $CO_2$ nozzle (i.e. double cryogenic expansion) is clearly evident in respect to the use of spiralling flow $CO_2$ alone (i.e. single cryogenic expansion). For clarity, we report here below the direct comparison in Table 9.

Table 9 Comparison of results of example 14 and comparative example 15 on water removal efficiency

| Example | TEST | $PN_2$ mixer (bar) | $PN_2$ (bar) | $CO_2$ flow pattern | %$H_2$Oi | %$H_2$Of | Water removal efficiency (%) |
|---|---|---|---|---|---|---|---|
| | DE042 | 1.5 | 2.0 | Swirl | 10.0 | 0.9 | 91.35 |
| 14 | DE043 | 1.5 | 2.0 | Swirl | 20.0 | 8.2 | 59.00 |
| | DE044 | 1.5 | 2.0 | Swirl | 30.0 | 14.0 | 53.00 |
| | DE007 | No | 3.5 | Swirl | 10 | 2.2 | 78.0 |
| 15 | DE008 | No | 3.5 | Swirl | 20 | 10.0 | 50.0 |
| | DE009 | No | 3.5 | Swirl | 30 | 16.0 | 46.7 |

**Example 17.** *Analysis of particles morphology. Microspheronization effect induced by the swirl flow $CO_2$ nozzle*

The morphology of the microparticles obtained in Comparative Example 15 (sample DE 007) was compared with that of the microparticles obtained in Comparative Example 16 (DE 052) by optical microscopy, see Figure 11. Microspheres produced by swirling $CO_2$ flow (sample DE 007) show a more spherical and regular morphology compared to sample DE 052, which shows irregular morphology with small filaments and elongated spheroidal microparticles. Ultimately, the use of $CO_2$ swirl nozzle allows a more efficient water removal and the production of spherically shaped micronized powders, less sticky and more flowable.

**Comparative Example 18.** *Microparticles production and water elimination capacity - effect of preatomization (static mixer configuration) combined with* $CO_2$ *cone flow and*

**Comparative Example 19.** *Microparticles production and water elimination capacity using configuration with product capillary flow nozzle and CO$_2$ cone flow*

In these examples we describe how the configuration with static mixer (preatomization) in combination with cone CO$_2$ flow does not have a relevant effect on water removal efficiency. The same formulation as described in Example 11 (Gelucire 50/13- Compritol 888 in a 15:85 ratio) was used in these sets of experiments. Process conditions and formulations are as follows. Product vessel temperature: 80.0 °C; Product line temperature: 80.0 °C; Product nozzle temperature: 85.0 °C; Product nozzle diameter: 0.85 mm; Product pressure (N$_2$): 2.0 bar; Pressure CO$_2$: 55 bar; CO$_2$ nozzle diameter (0.5 mm - cone flow nozzle). The results are provided in Table 10.

Table 10. Effect of the static mixer (preatomization) configuration in combination with CO$_2$ cone nozzle on water removal efficiency

| Example | TEST | PN$_2$ (bar) | PN$_2$ static mixer (bar) | %H$_2$Oi | %H$_2$Of | Water removal efficiency (%) |
|---------|------|--------------|---------------------------|----------|----------|------------------------------|
|         | DE039 | 2.0 | 1.5 | 10 | 5.4 | 46.0 |
| 18      | DE040 | 2.0 | 1.5 | 20 | 11.3 | 43.5 |
|         | DE041 | 2.0 | 1.5 | 30 | 18.3 | 39.0 |
|         | DE052 | 3.5 | NO | 10 | 4.5 | 53.0 |
| 19      | DE053 | 3.5 | NO | 20 | 11.8 | 41.0 |
|         | DE054 | 3.5 | NO | 30 | 17.2 | 42.7 |

The results gathered in Table 10 indicate that, if a cone CO$_2$ flow pattern is used, the effect of preatomization (static mixer in the equipment configuration) on water removal efficiency is lower, and practically not relevant.

**Comparative Example 20.** *Analysis of particles morphology. Lack of microspheronization effect when hollow cone CO$_2$ nozzle is used*

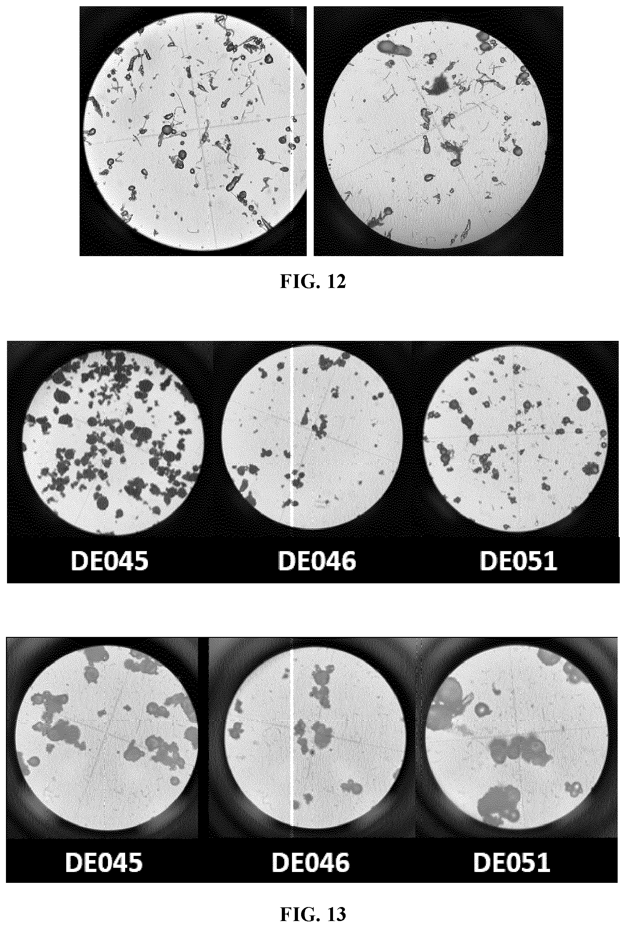

The morphology of the microparticles obtained in Comparative Examples 18 and 19 was compared by optical microscopy, see Figure 12. Microparticles appearance and size look similar in both examples 18 and 19. Particle morphology was irregular, with predominance of elongated particles, irregular shapes and wide size distribution, confirming that the preatomization alone cannot produce regular particles, and that no microspheronization effect is obtained unless the static mixer configuration is used in combination with a spiralling CO$_2$ flow nozzle.

**Example 21:** *Microparticles production and water removal capacity combining preatomization by static mixer with CO$_2$ swirling flow pattern: double cryogenic expansion. Testing different formulations*

In this example, the impact of different excipients and formulations (identified as DE045, DE046 and DE051, in Table 11) on water removal during cryospraying was tested. The equipment configuration includes the static mixer for preatomization, combined with the spiralling flow CO$_2$ nozzle. The formulations tested include two compositions containing stearic acid (SA), directly emulsified with water at 10% w/w and 20% w/w, so that stearic acid content before spraying is 90% and 80% respectively, and one blend of stearic acid and Gelucire 50/13 in a 70:30 ratio, emulsified with 25% water, so that the composition before spraying comprises 25% w/w water, 52,5% w/w stearic acid and 22.5 % w/w Gelucire 50/13. Process conditions. Product vessel temperature: 80.0 °C; Product line temperature: 80.0 °C; Product nozzle temperature: 85.0 °C; Product nozzle diameter: 0.85 mm; Product pressure (N$_2$): 2.0 bar; Pressure CO$_2$: 55 bar; CO$_2$ nozzle diameter (0.5 mm - spiraling flow nozzle).

Table 11. Effect of formulation on water removal using the static mixer and CO$_2$ swirl nozzle with mixtures of SA and water, and SA-G50/13 and water

| TEST | %SA | %G50/13 | %H$_2$Oi | %H$_2$Of | Water removal efficiency (%) |
|------|-----|---------|----------|----------|------------------------------|
| DE045 | 80 | 0 | 20.0 | 7.2 | 64.0 |
| DE046 | 90 | 0 | 10.0 | 1.9 | 81.5 |
| DE051 | 52.5 | 22.5 | 25.0 | 14.5 | 42.0 |

In all cases it was possible to produce microspheres in powder form. As expected, water removal was higher in the more hydrophobic formulations, while the composition containing the highly hydrophilic G50/13 tends to retain more water in the particles. Optical microscopy images (Figure 13) show microparticles with a well-defined spherical morphology and quite narrow particles size distribution, independently from the initial water content in the emulsion, and from the final residual moisture in the powders. The results of the example confirm the advantage of combining the static mixer for preatomization with the spiralling flow $CO_2$ nozzle in the equipment configuration, so to exploit the double cryogenic expansion and the $CO_2$ microspheronization effect described in the invention.

**Example 22.** *Microparticles production and water removal capacity according to the invention. Combining preatomization (static mixer in product line) with $CO_2$ swirling flow pattern. Initial water content in formulations up to 50% w/w and*

**Comparative Example 23.** *Microparticles preparation and water removal capacity without static mixer configuration with $CO_2$ swirling flow pattern. Initial water content in formulations up to 50% w/w*

Experiments on ternary mixtures containing up to about 40% water were processed at similar conditions, in a configuration with static mixer plus swirling flow $CO_2$ nozzle, and, in comparison, with swirling flow $CO_2$ nozzle but without static mixer in the product line. A Gelucire 50/13: Compritol 888 formulation at 15:85 fixed ratio was prepared and emulsified with different amounts of water at 10%, 20%, 30%, and 40% in weight. Therefore formulated compositions contained respectively 13.5%, 12%, 10.5%, and 9% by weight in Gelucire 50/13, and 76,5% , 68%, 59.5%, and 51%, by weight of Compritol 888 before spraying Equipment operational conditions were set as previously described in Example 14, and as described in Table 12. The $CO_2$ nozzle diameter was 0.5 mm, producing the spiraling flow pattern.

Table 12. Effect on water removal using the static mixer and $CO_2$ swirl nozzle (double cryogenic expansion) starting from formulations with high water content up to 45%)

| Ex. | TEST | PN$_2$ (bar) | PN$_2$ mixer (bar) | CO$_2$ flow pattern | CO$_2$ pressure (bar) | %H$_2$Oi | %H$_2$Of | Water removal efficiency (%) |
|---|---|---|---|---|---|---|---|---|
| | DE042 | 2.0 | 1.5 | Swirl | 56 | 10.0 | 0.9 | 91.35 |
| | DE043 | 2.0 | 1.5 | Swirl | 57 | 20.0 | 8,2 | 59.00 |
| 22 | DE044 | 2.0 | 1.5 | Swirl | 55 | 30.0 | 14.0 | 53.00 |
| | DE049 | 2.0 | 1.5 | Swirl | 60 | 40.0 | 11.7 | 70.75 |
| 23 | DE055 | 2.0 | NO | Swirl | 56 | 40.0 | N/A* | N/A* |

\* N/A Microspheres could not be produced at these conditions

Results reported in Table 12 indicate that, when the initial water content in formulation is above 30%, microspheres in flowable powder form, and without aggregates, could be obtained only with the configuration combining the static mixer with the spiralling $CO_2$ flow nozzle (samples DE049 and DE050). On the other hand, it was observed that the spiralling $CO_2$ *flow per se* was not sufficient to remove enough water during spraying to obtain a micronized powder. In fact, the same compositions (DE055 and DE056) resulted in wet, aggregated and sticky powders, not viable as formulated microspheres. Therefore, we can confirm that unexpectedly the combination of preatomization by static mixer with the swirling-flow $CO_2$ nozzle is highly effective in removal of water in cryogenic conditions, and obtaining dry microspheres quite independently of the initial water content in the sprayed product. Thus, the double cryogenic expansion produced by the initial preatomization of product and the simultaneous interaction with the spiralling, expanding flow of $CO_2$ unexpectedly resulted the most effective process to obtain microspheres in powder form under cryogenic conditions.

**Example 24.** *Curcumin Formulations of microspheres with high content in curcuminoids*

Curcumin Formulations of microspheres with high content in curcuminoids were prepared according to the invention (Example 24a). One example of formulation composition is described in % weight as follows

| SET | Geleol (%) | Gelucire 50/13 (%) | Transcutol (%) | Curcuma extract (%) |
|---|---|---|---|---|
| CU007 | 45.0 | 15.0 | 15.0 | 25.0 |

The preparation is obtained by mixing Gelucire 50/13 with Transcutol at 55-60 °C, and separately melting Geleol at 70 °C. The curcuma extract is slowly added to the first mixture until an homogeneous dispersion is formed, the molten Geleol is the added to the mixture until the sample is homogeneous and ready to be processed and sprayed.

The processing conditions for microspheres preparation are described as follows:

| SET | $T_{pv}$ (°C) | $T_L$ (°C) | $T_n$ (°C) | $P_{N2}$ (bar) | $Ø_{noz}$(mm) | $ØCO_2$(mm) |
|---|---|---|---|---|---|---|
| CU007 | 70.0 | 70.0 | 85.0 | 2.0 | 0.85 | 0.5 |

$T_{pv}$: Product vessel temperature. $T_L$: Line temperature. $T_n$: Product nozzle temperature
$P_{N2}$: Nitrogen pressure. $Ø_{no:z}$ Product nozzle diameter. $ØCO_2$: Carbon dioxide nozzle diameter

The formulation was sprayed using a 0.85 mm capillary flow product nozzle, and a 0.5 diameter carbon dioxide nozzle (swirling / spiraling $CO_2$ flow).

In a comparative test example 24b, the same CU007 formulation was sprayed at the same experimental conditions, with the exception of the substitution of the 0.5 mm spiraling flow $CO_2$ nozzle with a hollow cone, $CO_2$ flow nozzle.

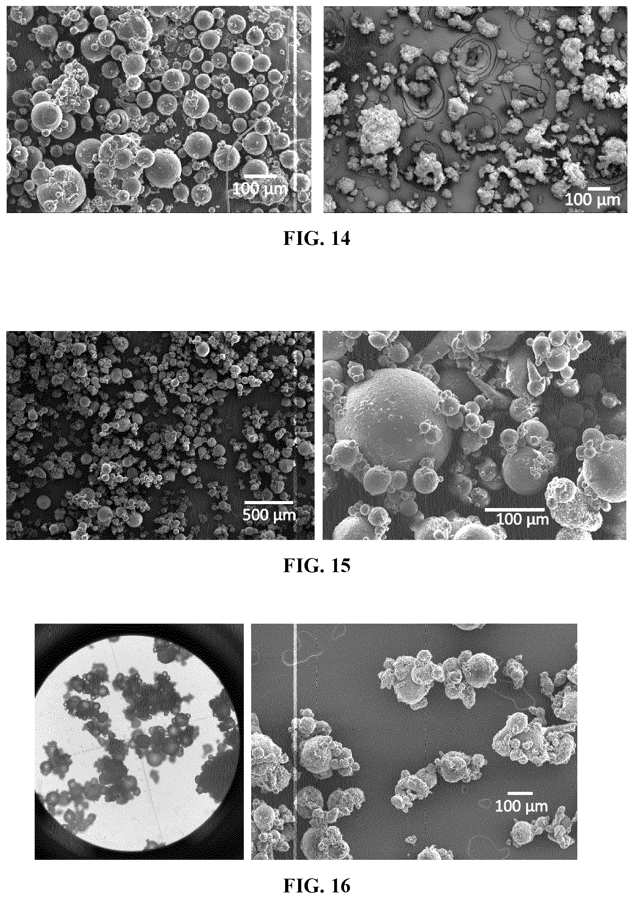

A direct comparison of size and morphology of CU007 samples of Example 24a and Comparative Example 24b, performed by SEM, is reported in Figure 14. It is clear how microspheres produced according to Example 24a are very spherical microparticles with homogeneous smooth surface and a narrower particles size distribution can be obtained.

**Example 25.** *Formulations of microspheres with high content in lycopene in sunflower oil*

Formulations of microspheres with high content in lycopene in sunflower oil (Lycovit ® D10, BASF, nominal Lycopene content 10%) were prepared using a 0.5 mm diameter swirling flow nozzle for $CO_2$. Two examples of formulation with lycopene are detailed in the following tables.

| SET | Compritol 888 (%) | Gelucire 50/13 (%) | Geleol (glyceril monostearate) | TPGS | Lycovit® (%) |
|---|---|---|---|---|---|
| LY017 | 15 | 15 | 35 | 10 | 25.0 |
| LY022 | 14.5 | 7 | 32.5 | 6 | 40.0 |

To prepare the formulations, lycopene extract is slowly added to a previously molten mixture of Gelucire, Geleol and TPGS at 55°C kept away from light, until it reaches a homogeneous appearance; to the mixture is then slowly added the molten Compritol 888, until an homogeneous monophasic mixture is obtained. The mix is then sprayed at the following process conditions.

| SET | $T_{pv}$ (°C) | $T_L$ (°C) | $T_n$ (°C) | $P_{N2}$ (bar) | $Ø_{noz}$(mm) | $ØCO_2$(mm) |
|---|---|---|---|---|---|---|
| LY017-LY022 | 75.0 | 80.0 | 85.0 | 2.5 | 0.5 | 0.5 |

$T_{pv}$: Product vessel temperature. $T_L$: Line temperature. $T_n$: Product nozzle temperature
$P_{N2}$: Nitrogen pressure. $Ø_{no:z}$ Product nozzle diameter. $ØCO_2$: Carbon dioxide nozzle diameter

The formed microparticles were recovered and characterized for particles size, morphology, and lycopene content. SEM images on lycopene micronized powders (Figure 15) showed small microparticles, spherical in shape (less than 100 $\mu$m in most cases) with a compact matrix-like structure and a smooth surface. These results show that the double expansion cryopsraying process can be applicable to produce spherically shaped, homogeneously distributed microparticles even when used with complex, natural extracts at high amounts.

Total lycopene content and its isomers were determined in the microparticles and in the starting material by HPLC (UV-DAD) analysis method. Assays in formulated microparticles, and in Lycovit® starting material were determined by an isocratic HPLC method, using an Agilent 1200 HPLC - DAD equipment. Concentrations of lycopene cis-form, lycopene trans-form, and total lycopene in the samples were determined Column Develosil; 250 mm x 4,6 mm x 5 $\mu$m RP, mobile phase methanol : IPA : THF (30:30:35) % (v/v). Lycopene was detected at 472 nm wavelength vs. lycopene standard Experimental assay values of lycopene are in good agreement with the theoretical values. In addition, HPLC analysis shows that the *trans* isomer is the predominant form in the tested microparticles as in the starting material, suggesting that cryospraying processing does not affect the stability of the active ingredient.

| Microspheres Sample | Total Lycopene (% load) | % 5-cis | % all-trans |
|---|---|---|---|
| LY017 | 2.064 | 6.42 | 91.58 |
| LY022 | 4.820 | 8.30 | 91.70 |
| Lycovit® D 10 | 11.796 | 3.16 | 94.09 |

**Example 26.** *Microspheres containing complex natural extract from wine lees*

Wine lees extracts present quite a challenge to formulation and subsequent microspheres formation by $CO_2$ cryospraying according to the invention, since the material to be formulated is a relatively thick suspension, obtained by wet milling, and containing about 20% of insoluble, micronized solids, in a hydroalcoholic solution of polyphenols and other soluble components, with a high water-ethanol content. Extracts from wine lees were provided by Lurederra Technological Center (Spain), and prepared as follows: wine lees were collected and vacuum filtered to a pasty material; subsequently, wine lees extracts were obtained by ultrasound processing and wet milling, to produce an homogeneous, pasty material which was used for the microspheres preparation.

In this example, three formulations with wine lees extracts content up to about 26% were prepared, according to the following excipient compositions.

| Formulation | C888 (wt%) | Geleol (wt%) | G50/13 (wt%) | Labrasol (wt%) | MCT (wt%) | Wine lees extract (wt%) |
|---|---|---|---|---|---|---|
| VI001 | 12.6 | 24.1 | 15.3 | 10 | 5 | 15 |
| VI002 | 10 | 39 | 11 | 13.3 | 6.7 | 19.9 |
| VI003 | 7 | 34 | 7.9 | 17 | 8.5 | 25.5 |

Microspheres were prepared using a 1.0 mm diameter swirling flow nozzle to the $CO_2$ so that almost optimal conditions for cryospraying and water elimination could be applied, considering that the starting material (wine lees extract) has 80% of water content, as determined by Karl-Fisher analysis.

| SET | $T_{pv}$ ($^0$C) | $T_L$($^0$C) | $T_n$($^0$C) | $P_{N2}$ (bar) | $Ø_{noz}$(mm) | $ØCO_2$(mim) |
|---|---|---|---|---|---|---|
| VI001;002;003 | 65.0 | 75.0 | 65.0 | 2.5 | 0.85 | 1.0 |

$T_{pv}$: Product vessel temperature. $T_L$: Line temperature. $T_n$: Product nozzle temperature
$P_{N2}$: Nitrogen pressure. $Ø_{no:z}$ Product nozzle diameter. $ØCO_2$: Carbon dioxide nozzle diameter

It was possible to spray the formulations, and to obtain small microspheres with homogeneous distributions. Microspheres appeared macroscopically as a homogeneous, flowable fine powder. Optical microscopy (100x) and SEM images (Figure 16) confirm that the powder is composed by small spherical particles that the wine lees were homogeneously dispersed in the matrix, and microspheres presented a compact matrix-like structure

Microspheres were characterized for their residual water content, for total polyphenols (by UV method, (as gallic acid equivalents), and for total sugars content (as glucose equivalents). Total polyphenols content was measured by UV-VIS method (Folin-Ciocolteau method), against a calibration curve at 765 nm maximum absorbance, and expressed as gallic acid equivalents. Total saccharides content were determined by treating the samples with the phenol-sulfuric method, measured by UV VIS and expressed as glucose equivalents against a glucose calibration curve. Residual water content in the various microspheres formulations are reported herebelow, in comparison with the wine lees extract starting material. The key results obtained in the microspheres are summarized in the following table.

| Microspheres Formulation | Theoretical content (wine lees extract) | Water conten t | Total Polyphenol s (g/Kg) | Total Saccharide s (g/Kg) |
|---|---|---|---|---|
| VI001 | 15 % | 8.4% | 8.18 | 2.45 |
| VI002 | 19.9 % | 6.4 % | 18.85 | 12.21 |
| VI003 | 25.5 % | 13.2 % | 11.89 | 29.67 |

(continued)

| Microspheres Formulation | Theoretical content (wine lees extract) | Water conten t | Total Polyphenol s (g/Kg) | Total Saccharide s (g/Kg) |
|---|---|---|---|---|
| Wine Lees (starting material) | - | 79.9 % | 13.0 | 30.6 |

Remarkably and unexpectedly, it was possible to produce microspheres containing the whole natural product originated from wine lees. The microspheronization process of the invention allowed to eliminate great amounts of water, or water-ethanol mixtures directly during spraying. The process allowed to maintain the biomolecules of interest within the particles matrices, and at a surprisingly high concentration up to 2.97% in total sugars content, and to 1.9 % in polyphenols, equal or superior to the starting material (3.0% and 1.30% respectively).

**Example 27. *Effect of combination of static mixer and $CO_2$ swirling flow on particles size of microspheres***

Microspheres prepared as previously described in Example 22 (sample DE042, Table 12) and Comparative Example 19 (sample DE054, Table 10) were analyzed for particles size distribution with a Malvern Morphology 4 analyzer, applying a dry dispersion protocol, described in the methods section, that allows to directly analyze the samples in powder form. Samples DE042 and DE054 have the same excipients composition (Compritol 888 and Gelucire 50/13 at 85:15 ratio), while they differ for the process conditions applied in their manufacturing. DE042 was prepared using the equipment configuration according to the invention, comprising the use of the static mixer and the swirling $CO_2$ flow, while DE054 was prepared without static mixer in the product line, and using a hollow cone $CO_2$ flow.

Table 13. Cumulative particles size distribution (volumetric) of microspheres produced with static mixer and swirling $CO_2$ flow, and without static mixer and hollow cone $CO_2$ flow configurations (example 22 and comparative example 19 respectively)

| Sample | Product line | $CO_2$ line | Dx (10) $\mu$m | Dx (50) $\mu$m | Dx (90) $\mu$m |
|---|---|---|---|---|---|
| Example 22 DE042 | Static Mixer | Swirl Flow | 25.2 | 63.2 | 137.4 |
| Example 19 DE054 | Capillary | Hollow Cone | 45.3 | 107.1 | 273.8 |

Results reported in Table 13 show that, for the same compositions, the equipment configuration according to the invention clearly facilitates the formation of smaller microparticles.

**Example 28. *Effect of combination of static mixer and $CO_2$ swirling flow on particles size of microspheres***

Microspheres prepared as previously described in Example 4 (samples SMX003 and SMX004) were analyzed for particles size distribution with a Malvern Morphology 4 analyzer, applying the dry dispersion protocol directly on the samples in powder form. Samples SMX003 and SMX004 have the same excipients composition (100% Glyceryl tristearate), and were prepared using the equipment configuration according to the invention, comprising the use of the static mixer in the product line and the swirling $CO_2$ flow in the liquid $CO_2$ line. They differed only for the $CO_2$ nozzle diameter, being 1.0 mm and 0.5 mm respectively.

Table 14. Cumulative particles size distribution (numeric) of microspheres produced with static mixer and swirling $CO_2$ flow configuration, demonstrating the formation of very small particles

| Sample | Product line | $CO_2$ line | $CO_2$ nozzle | Dx (10) $\mu$m | Dx (50) $\mu$m | Dx (90) $\mu$m |
|---|---|---|---|---|---|---|
| Example 4 SMX003 | Static Mixer | Swirl Flow | 1 mm | 0.9 | 1.3 | 3.3 |
| Example 4 SMX 004 | Static Mixer | Swirl flow | 0.5mm | 1.0 | 2.5 | 24.6 |

Particles size distributions (numeric) for SMX003 and SMX004 demonstrate that the double cryogenic expansion process according to the invention allows the formation of microspheres with very small particles sizes and quite narrow distributions (Table 14).

**Example 29.** *Effect of combination of static mixer and $CO_2$ swirling flow on circularity (sphericity) of microspheres: microspheronization effect*

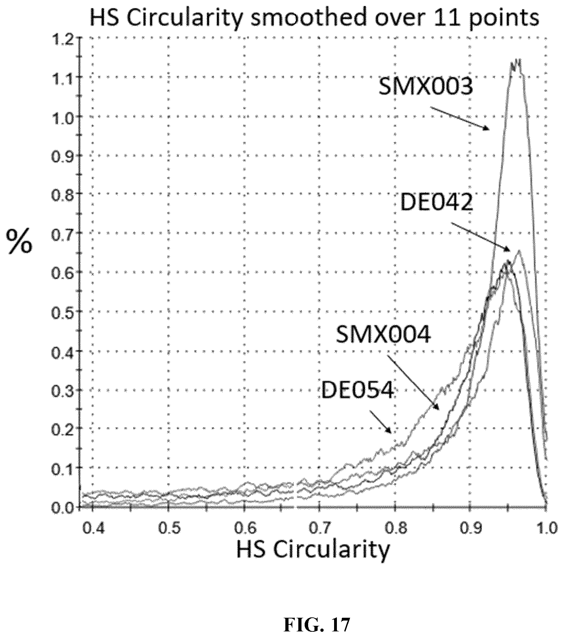

In figure 17, circularity distribution of 4 different microspheres samples (SMX003 and SMX004, from example 4; DE042 from example 22; DE054 from comparative example 19) are plotted. It is evident from the results that sharper distributions close to 1.0 circularity are obtained when microparticles are produced with the configuration according to the invention (SMX003; SMX004; DE042), as compared to sample DE054.

## Claims

1.  Process for the manufacture of solid microparticles comprising:

    a) providing a fluid mixture comprising one or more molecules of interest, one or more compounds selected from the group consisting of lipids, lipid-polymer compounds and polymers, and optionally water,
    b) mixing the fluid mixture of step a) with a desiccating gas and/or plasticizing gas to provide a dispersion of the fluid mixture of step a) and the desiccating gas and/or plasticizing gas,
    c) ejecting the dispersion obtained in step b) through a nozzle thereby creating a capillary or preatomized flow, and
    d) contacting the capillary or preatomized flow obtained in step c) with an expanding liquid or dense $CO_2$ swirling or spiraling flow to obtain the solid microparticles.

2.  Process according to claim 1, wherein the one or more lipids, lipid-polymer compounds and polymers of the fluid mixture of step a) is selected from the group consisting of mono-, di- and tri-glycerides of fatty acids, polyoxylglycerides and TPGS.

3.  Process according to any one of the preceding claims, wherein the fluid mixture of step a) is in the form of an emulsion, microemulsion, nanoemulsion, suspension, solution, concentrated solution or heterogeneous mixture.

4.  Process according to any one of the preceding claims, wherein the molecule of interest is hygroscopic.

5.  Process according to any one of the preceding claims, wherein the desiccating gas and/or plasticizing gas of step b) is selected from the group consisting of nitrogen, $CO_2$ and a mixture thereof, preferably nitrogen.

6.  Process according to any one of the preceding claims, wherein the desiccating gas and/or plasticizing gas of step b) is provided at a pressure of from 0.2 to 16 bar, preferably from 1 to 5 bar, more preferably from 1 to 2 bar.

7.  Process according to any one of the preceding claims, wherein the expanding liquid or dense $CO_2$ of step d) is provided at a pressure before expansion of from 15 to 70 bar, preferably from 45 to 60 bar.

8.  Process according to any one of the preceding claims, wherein the flows that are contacted in step d) are ejected at an angle of from 0° to 90° with respect to each other, preferably from 15° to 50°, more preferably from 30° to 45°.

9.  Process according to any one of the preceding claims, wherein the fluid mixture of step a) is provided at a temperature of from 4 °C to 250 °C, preferably of from 30 °C to 95 °C and more preferably from 50 °C to 85 °C step b) is carried out at a temperature of from 20 °C to 250 °C, preferably of from 30 °C to 95 °C and more preferably from 50 °C to 85 °C , step c) is carried out at a temperature of from 20 °C to 250 °C, preferably of from 30 °C to 95 °C and more preferably from 45 °C to 85 °C , and/or step d) is carried out at a temperature of from -20 °C to +35 °C.

10. Process according to any one of the preceding claims, wherein the process is performed in batch or continuous.

11. Apparatus for performing the process according to any one of the preceding claims, the apparatus comprising:

    i) a first vessel for providing the fluid mixture comprising the one or more molecules of interest, the one or more compounds selected from the group consisting of lipids, lipid-polymer compounds and polymers, and optionally water,
    ii) a source of the desiccating gas and/or plasticizing gas,
    iii) a device for mixing the fluid mixture comprising the one or more molecules of interest, the one or more compounds selected from the group consisting of lipids, lipid-polymer compounds and polymers and optionally water, with the desiccating gas and/or plasticizing gas to provide the dispersion of the fluid mixture and the desiccating gas and/or plasticizing gas,

iv) means for conveying the fluid mixture of the first vessel i) to the device iii),

v) means for conveying the desiccating gas and/or plasticizing gas from the source ii) to the device iii),

vi) a second vessel for microparticle formation,

vii) one or more nozzles for creating a capillary or preatomized flow of the dispersion of the fluid mixture and the desiccating gas and/or plasticizing gas located in the upper part of the second vessel vi),

viii) means for conveying the dispersion of the fluid mixture and the desiccating gas and/or plasticizing gas of the device iii) to the one or more nozzles vii),

ix) a source of the liquid or dense $CO_2$,

x) a nozzle for creating a swirling or spiraling flow of liquid or dense $CO_2$ located in the upper part of the second vessel vi),

xi) means for conveying the liquid or dense $CO_2$ from the source ix) to the nozzle x), and wherein the one or more nozzles vii) are positioned at a distance with respect to nozzle x) of from 0.1 to 500 mm, preferably from 5 to 50 mm.

12. The apparatus according to claim 11, wherein the device iii) is a static mixer and/or wherein the second vessel vi) is a spray tower.

13. The apparatus according to any one of claims 11 or 12, wherein the one or more nozzles vii) are at an angle of from 0° to 90° with respect to the nozzle x), preferably from 15° to 50°, more preferably from 30 to 45°; and/or wherein the one or more nozzles vii) are positioned with respect to nozzle x) to provide a distance of incidence from 0 mm to 300 mm, preferably from 5 to 50 mm and more preferably from 20 to 30 mm.

14. The apparatus according to any one of claims 11 to 13, wherein nozzle x) is positioned vertically.

15. The apparatus according to any one of claim 11 to 14, wherein the first vessel i), the device ii), the means iv), the means viii), the one or more nozzles vii) comprise heating means.

**Patentansprüche**

1. Verfahren zur Herstellung fester Mikropartikel, umfassend:

a) das Bereitstellen eines Fluidgemisches, umfassend ein oder mehrere Moleküle von Interesse, eine oder mehrere Verbindungen, ausgewählt aus der Gruppe, bestehend aus Lipiden, Lipid-Polymer-Verbindungen und Polymeren, und gegebenenfalls Wasser,

b) das Mischen des Fluidgemisches aus Schritt a) mit einem Trocknungsgas und/oder einem Plastifizierungsgas, um eine Dispersion des Fluidgemisches aus Schritt a) und des Trocknungsgases und/oder Plastifizierungsgases bereitzustellen,

c) das Ausstoßen der in Schritt b) erhaltenen Dispersion durch eine Düse, wodurch ein kapillarer oder vorzerstäubter Strom erzeugt wird, und

d) das Inkontaktbringen des in Schritt c) erhaltenen kapillaren oder vorzerstäubten Stroms mit einem expandierenden flüssigen oder dichten $CO_2$-Wirbel- oder Spiralstrom, um die festen Mikropartikel zu erhalten.

2. Verfahren nach Anspruch 1, wobei das eine oder die mehreren Lipide, Lipid-Polymer-Verbindungen und Polymere des Fluidgemisches aus Schritt a) ausgewählt sind aus der Gruppe, bestehend aus Mono-, Di- und Triglyceriden von Fettsäuren, Polyoxylglyceriden und TPGS.

3. Verfahren nach einem der vorstehenden Ansprüche, wobei das Fluidgemisch aus Schritt a) in Form einer Emulsion, Mikroemulsion, Nanoemulsion, Suspension, Lösung, konzentrierten Lösung oder eines heterogenen Gemisches vorliegt.

4. Verfahren nach einem der vorstehenden Ansprüche, wobei das Molekül von Interesse hygroskopisch ist.

5. Verfahren nach einem der vorstehenden Ansprüche, wobei das Trocknungsgas und/oder Plastifizierungsgas aus Schritt b) ausgewählt ist aus der Gruppe, bestehend aus Stickstoff, $CO_2$ und einem Gemisch davon, vorzugsweise Stickstoff.

6. Verfahren nach einem der vorstehenden Ansprüche, wobei das Trocknungsgas und/oder Plastifizierungsgas aus Schritt b) bei einem Druck von 0,2 bis 16 bar, vorzugsweise von 1 bis 5 bar, noch bevorzugter von 1 bis 2 bar, bereitgestellt wird.

7. Verfahren nach einem der vorstehenden Ansprüche, wobei das expandierende flüssige oder dichte $CO_2$ aus Schritt d) vor der Expansion mit einem Druck von 15 bis 70 bar, vorzugsweise von 45 bis 60 bar, bereitgestellt wird.

8. Verfahren nach einem der vorstehenden Ansprüche, wobei die in Schritt d) in Kontakt gebrachten Ströme in einem Winkel von 0° bis 90°, vorzugsweise von 15° bis 50°, noch bevorzugter von 30° bis 45°, zueinander ausgestoßen werden.

9. Verfahren nach einem der vorstehenden Ansprüche, wobei das Fluidgemisch aus Schritt a) bei einer Temperatur von 4 °C bis 250 °C, vorzugsweise von 30 °C bis 95 °C und noch bevorzugter von 50 °C bis 85 °C, bereitgestellt wird, Schritt b) bei einer Temperatur von 20 °C bis 250 °C, vorzugsweise bei einer Temperatur von 30 °C bis 95 °C und noch bevorzugter bei einer Temperatur von 50 °C bis 85 °C, durchgeführt wird, Schritt c) bei einer Temperatur von 20 °C bis 250 °C, vorzugsweise von 30 °C bis 95 °C und noch bevorzugter von 45 °C bis 85 °C, durchgeführt wird und/oder Schritt d) bei einer Temperatur von -20 °C bis +35 °C durchgeführt wird.

10. Verfahren nach einem der vorstehenden Ansprüche, wobei das Verfahren im Batch oder kontinuierlich durchgeführt wird.

11. Vorrichtung zur Durchführung des Verfahrens gemäß einem der vorstehenden Ansprüche, wobei die Vorrichtung umfasst:

  i) einen ersten Behälter zur Bereitstellung des Fluidgemisches, umfassend das eine oder die mehreren Moleküle von Interesse, die eine oder die mehreren Verbindungen, ausgewählt aus der Gruppe, bestehend aus Lipiden, Lipid-Polymer-Verbindungen und Polymeren, und gegebenenfalls Wasser,
  ii) eine Quelle für das Trocknungsgas und/oder Plastifizierungsgas,
  iii) eine Vorrichtung zum Mischen des Fluidgemisches, umfassend das eine oder die mehreren Moleküle von Interesse, die eine oder die mehreren Verbindungen, ausgewählt aus der Gruppe, bestehend aus Lipiden, Lipid-Polymer-Verbindungen und Polymeren, und gegebenenfalls Wasser, mit dem Trocknungsgas und/oder Plastifizierungsgas, um die Dispersion des Fluidgemisches und des Trocknungsgases und/oder Plastifizierungsgases bereitzustellen,
  iv) Mittel zum Befördern des Fluidgemisches aus dem ersten Behälter i) zu der Vorrichtung iii),
  v) Mittel zum Befördern des Trocknungsgases und/oder Plastifizierungsgases aus der Quelle ii) zu der Vorrichtung iii),
  vi) einen zweiten Behälter zur Bildung von Mikropartikeln,
  vii) eine oder mehrere Düsen zum Erzeugen eines kapillaren oder vorzerstäubten Stroms der Dispersion des Fluidgemisches und des Trocknungsgases und/oder Plastifizierungsgases, die sich im oberen Teil des zweiten Behälters vi) befinden,
  viii) Mittel zum Befördern der Dispersion des Fluidgemisches und des Trocknungsgases und/oder Plastifizierungsgases von der Vorrichtung iii) zu der einen oder den mehreren Düsen vii),
  ix) eine Quelle für flüssiges oder dichtes $CO_2$,
  x) eine Düse zur Erzeugung eines wirbelnden oder spiralförmigen Stroms von flüssigem oder dichtem $CO_2$, die sich im oberen Teil des zweiten Behälters vi) befindet,
  xi) Mittel zum Befördern des flüssigen oder dichten $CO_2$ von der Quelle ix) zu den Düsen x), und wobei die eine oder mehreren Düsen vii) in einem Abstand von 0,1 bis 500 mm, vorzugsweise von 5 bis 50 mm, zu der Düse x) angeordnet sind.

12. Vorrichtung nach Anspruch 11, wobei die Vorrichtung iii) ein statischer Mischer ist und/oder wobei der zweite Behälter vi) ein Sprühturm ist.

13. Vorrichtung nach einem der Ansprüche 11 oder 12, wobei die eine oder mehreren Düsen vii) in einem Winkel von 0° bis 90° zu der Düse x), vorzugsweise von 15° bis 50°, noch bevorzugter von 30 bis 45°, angeordnet sind; und/oder wobei die eine oder mehreren Düsen vii) in Bezug auf die Düse x) so positioniert sind, dass sie einen Auftreffabstand von 0 mm bis 300 mm, vorzugsweise von 5 bis 50 mm und noch bevorzugter von 20 bis 30 mm, bereitstellen.

14. Vorrichtung nach einem der Ansprüche 11 bis 13, wobei die Düse x) vertikal positioniert ist.

**15.** Vorrichtung nach einem der Ansprüche 11 bis 14, wobei der erste Behälter i), die Vorrichtung ii), die Mittel iv), die Mittel viii) und die eine oder mehreren Düsen vii) eine Heizmittel umfassen.

**Revendications**

1. Procédé pour la fabrication de microparticules solides, comprenant :

   a) fournir un mélange fluide comprenant une ou plusieurs molécules d'intérêt, un ou plusieurs composés choisis parmi le groupe constitué de lipides, de composés lipides-polymères et de polymères, et facultativement d'eau,
   b) mélanger le mélange fluide de l'étape a) avec un gaz desséchant et/ou un gaz plastifiant pour assurer une dispersion du mélange fluide de l'étape a) et du gaz desséchant et/ou du gaz plastifiant,
   c) éjecter la dispersion obtenue à l'étape b) à travers une buse en créant ainsi un écoulement capillaire ou pré-atomisé, et
   d) mettre en contact le flux capillaire ou pré-atomisé obtenu à l'étape c) avec un écoulement tourbillonnant ou en spirale de $CO_2$ liquide ou dense en expansion pour obtenir les microparticules solides.

2. Procédé selon la revendication 1, dans lequel le un ou plusieurs lipides, composés lipides-polymères et polymères du mélange fluide de l'étape a) sont choisis parmi le groupe constitué des mono-, di- et tri-glycérides d'acides gras, polyoxyglycérides et TPGS.

3. Procédé selon l'une quelconque des revendications précédentes, dans lequel le mélange fluide de l'étape a) se présente sous la forme d'une émulsion, d'une micro-émulsion, d'une nano-émulsion, d'une suspension, d'une solution, d'une solution concentrée ou d'un mélange hétérogène.

4. Procédé selon l'une quelconque des revendications précédentes, dans lequel la molécule d'intérêt est hygroscopique.

5. Procédé selon l'une quelconque des revendications précédentes, dans lequel le gaz desséchant et/ou le gaz plastifiant de l'étape b) est choisi parmi le groupe constitué de l'azote, du $CO_2$ et d'un mélange de ceux-ci, de préférence l'azote.

6. Procédé selon l'une quelconque des revendications précédentes, dans lequel le gaz desséchant et/ou le gaz plastifiant de l'étape b) est fourni à une pression de 0,2 à 16 bar, de manière préférée de 1 à 5 bar, de manière plus préférée de 1 à 2 bar.

7. Procédé selon l'une quelconque des revendications précédentes, dans lequel le $CO_2$ liquide ou dense en expansion de l'étape d) est fourni à une pression avant expansion de 15 à 70 bar, de préférence de 45 à 60 bar.

8. Procédé selon l'une quelconque des revendications précédentes, dans lequel les écoulements qui sont mis en contact à l'étape d) sont éjectés à un angle de 0° à 90° les uns par rapport aux autres, de manière préférée de 15° à 50°, de manière plus préférée de 30° à 45°.

9. Procédé selon l'une quelconque des revendications précédentes, dans lequel le mélange fluide de l'étape a) est fourni à une température de 4 °C à 250 °C, de manière préférée de 30 °C à 95 °C et de manière plus préférée de 50 °C à 85 °C, l'étape b) est réalisée à une température de 20 °C à 250 °C, de manière préférée de 30 °C à 95 °C et de manière plus préférée de 50 °C à 85 °C, l'étape c) est réalisée à une température de 20 °C à 250 °C, de manière préférée de 30 °C à 95 °C et de manière plus préférée de 45 °C à 85 °C et/ou l'étape d) est réalisée à une température de -20 °C à +35 °C.

10. Procédé selon l'une quelconque des revendications précédentes, dans lequel le procédé est mis en œuvre en discontinu ou en continu.

11. Appareil pour mettre en œuvre le procédé selon l'une quelconque des revendications précédentes, l'appareil comprenant :

   i) un premier réservoir pour fournir le mélange fluide comprenant une ou plusieurs molécules d'intérêt, un ou plusieurs composés choisis parmi le groupe constitué de lipides, de composés lipides-polymères et de polymères, et facultativement d'eau,

ii) une source de gaz desséchant et/ou de gaz plastifiant,

iii) un dispositif pour mélanger le mélange fluide comportant la ou les molécules d'intérêt, le ou les composés choisis parmi le groupe constitué de lipides, de composés lipides-polymères et de polymères et facultativement d'eau, avec le gaz desséchant et/ou le gaz plastifiant pour assurer la dispersion du mélange fluide et du gaz desséchant et/ou du gaz plastifiant,

iv) des moyens pour transférer le mélange fluide du premier réservoir i) vers le dispositif iii),

v) des moyens pour transférer le gaz desséchant et/ou le gaz plastifiant de la source ii) au dispositif iii),

vi) un second réservoir pour la formation de microparticules,

vii) une ou plusieurs buses pour créer un écoulement capillaire ou pré-atomisé de la dispersion du mélange fluide et du gaz desséchant et/ou du gaz plastifiant situés dans la partie supérieure du second réservoir vi),

viii) des moyens pour transférer la dispersion du mélange fluide et du gaz desséchant et/ou du gaz plastifiant du dispositif iii) vers la ou les buses vii),

ix) une source du $CO_2$ liquide ou dense,

x) une buse pour créer un écoulement tourbillonnant ou en spirale de $CO_2$ liquide ou dense, située dans la partie supérieure du second réservoir vi),

xi) des moyens pour transférer le $CO_2$ liquide ou dense de la source ix) à la buse x), et dans lequel la ou les buses vii) sont positionnées à une distance par rapport à la buse x) de 0,1 à 500 mm, de préférence de 5 à 50 mm.

12. Appareil selon la revendication 11, dans lequel le dispositif iii) est un mélangeur statique et/ou dans lequel le second réservoir vi) est une tour de pulvérisation.

13. Appareil selon l'une quelconque des revendications 11 ou 12, dans lequel la ou les buses vii) sont à un angle de 0° à 90° par rapport à la buse x), de manière préférée de 15° à 50°, de manière plus préférée de 30° à 45° ; et/ou dans lequel la ou les buses vii) sont positionnées par rapport à la buse x) pour fournir une distance d'incidence de 0 mm à 300 mm, de manière préférée de 5 à 50 mm et de manière plus préférée de 20 mm à 30 mm.

14. Appareil selon l'une quelconque des revendications 11 à 13, dans lequel la buse x) est positionnée verticalement.

15. Appareil selon l'une quelconque des revendications 11 à 14, dans lequel le premier réservoir i), le dispositif ii), les moyens iv), les moyens viii), la ou les buses vii) comportent des moyens de chauffage.

**FIG. 1**

**FIG. 2**

**FIG. 3**

CO$_2$ nozzle

Swirl effect

Product nozzle

Capillary flow

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

FIG. 14

FIG. 15

FIG. 16

**FIG. 17**

**EP 4 440 732 B1**

**REFERENCES CITED IN THE DESCRIPTION**

**Patent documents cited in the description**

- WO 2005053656 A1
- EP 1703965 B1
- US 2005051917 A1
- WO 2011159218 A1
- WO 2007028421 A
- WO 9922855 A
- WO 2005049192 A1
- EP 3106217 B1
- WO 2007097626 A1
- WO 2007065716 A2
- US 5851453 A
- US 6056791 A
- EP 2298286 B
- US 6630121 B1
- DE 007008009
- DE 0039040041
- DE 030031032
- DE 042043044